# EUROPÄISCHE PATENTSCHRIFT

(11) **EP 2 142 657 B1**
(45) Veröffentlichungstag und Bekanntmachung des Hinweises auf die Patenterteilung: **01.09.2010**
(21) Anmeldenummer: 08748997.7
(22) Anmeldetag: 18.04.2008
(51) Int. Cl.: C12P 7/26

(54) **VERWENDUNG DES PIGD-PROTEINS ZUR KATALYSE VON 1,4-ADDITIONEN VON 2-OXOALKANOATEN AN ALPHA, BETA-UNGESÄTTIGTE KETONE**
USE OF THE PIGD PROTEIN FOR CATALYZING 1,4-ADDITIONS OF 2-OXOALKANOATES TO ALPHA, BETA-UNSATURATED KETONES
UTILISATION DE LA PROTÉINE PIGD POUR LA CATALYSE D'ADDITIONS 1,4 DE 2-OXOALCANOATES À DES CÉTONES ALPHA, BÊTA-INSATURÉES

(30) Priorität: 09.05.2007 DE 102007021698
(43) Veröffentlichungstag der Anmeldung: 13.01.2010
(73) Patentinhaber: Albert-Ludwigs-Universität Freiburg, 79085 Freiburg (DE)
(72) Erfinder: MÜLLER, Michael, 79194 Gundelfingen (DE); DRESEN, Carola, 79104 Freiburg (DE); RICHTER, Michael, CH-9014 St.Gallen (CH)
(74) Vertreter: Keller, Günter
(86) Internationale Anmeldenummer: PCT/EP2008/003152
(87) Internationale Veröffentlichungsnummer: WO 2008/138450

(56) Entgegenhaltungen:
- WILLIAMSON, N.R. ET AL.: "Biosynthesis of the red antibiotic, prodigiosin, in Serratia: identification of a novel 2-methyl-3-n-amyl-pyrrole (MAP) assembly pathway, definition of the terminal condensing enzyme, and implications for undecylprodigiosin biosynthesis in Streptomyces" MOLECULAR MICROBIOLOGY, Bd. 56, Nr. 4, Mai 2005 (2005-05), Seiten 971-989, XP002497155 in der Anmeldung erwähnt
- DATABASE EMBL [Online] 7. Dezember 2004 (2004-12-07), HARRIS, A.K.P. ET AL.: "Putative uncharacterized protein pigD" XP002497156 Database accession no. Q5W251 in der Anmeldung erwähnt

### SEQUENCE LISTING

<110> universität Freiburg
<120> Verwendung des PigD-Proteins zur Katalyse von 1,4-,Additionen von
2-Oxoalkanoaten an a-, β-ungesättigte Ketone
<130> PigD
<160> 2
<170> PatentIn version 3.3
<210> 1
   <211> 903
   <212> PRT
   <213> serratia marcescens
<400> 1
   Met Arg Ala Ala Thr Ala Ala Cys Arg Asp Arg Arg Gly Leu Cys Arg
   1               5                   10                  15
   Ala Glu Phe Ala Arg Leu Ala Glu Ala Val Thr Pro Phe Trp Leu His
               20                  25                  30
   Lys Glu Leu Ile Met Thr Thr Leu Thr Gly Gln Ala Arg Leu Thr Asn
           35                  40                  45
   Ser Ala Ala Tyr Glu Gln Val Trp Gln Ala Glu Arg Gln Ala Cys Arg
       50                  55                  60
   Thr Asp Ala Asp Pro Asp Thr Leu Thr Val Gly Val Val Val Val Thr
   65                  70                  75                  80
   Arg Asn Pro Ala Phe Phe Gln Thr Gly Leu Ser Val Leu Asn Asp Ile
                   85                  90                  95
   Arg Asp Tyr Val Phe Asn Arg Val His Ile Gln Ser Glu Met Pro Leu
               100                 105                 110
   Lys Leu Leu Asp Leu Ala Ala Asp Ser Leu Tyr Leu Ala Ala Arg Glu
           115                 120                 125
   Lys Ala Leu His Phe Leu Lys Gly Gln Asn Lys Ala Ile Asn Val Arg
       130                 135                 140
   Ile Ile Gln Cys Ala Ser Leu Ala Glu Ala Thr Gly Lys Ile Ile Tyr
   145                 150                 155                 160
   Thr His Ala Leu Glu Gln Arg Pro Glu Phe His Leu Gly Met Leu Phe
                   165                 170                 175
   Tyr Asp Gln Thr Thr Pro Ala Gly Val Asp Asp Ser Ile Glu Gln Ile
               180                 185                 190
   Asp Arg Asp Leu Asp Ala Phe Tyr Ser Ala Leu Gln Arg Ser Gly Ile
   195 200 205
   Pro Ala Phe Tyr Thr Thr Phe Ser Thr Val Ala Phe Ile Arg Gln Leu
   210 215 220
   Arg Ser Pro Phe Arg Tyr Leu Pro Gln Gln Tyr Arg Glu Ile Val Arg
   225 230 235 240
   Ser Glu Asp Pro Ala Ile Phe Gln Thr Glu Leu Leu Cys Leu Trp Met
   245 250 255
   Asp Phe Phe Glu Met Asn Tyr Thr Asn Arg Arg Val Lys Pro Ile Gly
   260 265 270
   Ala Leu Ala Leu His Asn Thr Leu Gly Glu Gln Leu Ile Gln Phe Phe
   275 280 285
   Glu Arg Thr Ala Ala Glu Arg Trp Leu Val Ser Tyr Tyr Thr Gly Ser
   290 295 300
   Ile Ile Ser Asn Leu Ile Gly Tyr Leu Asp Arg His Ala Glu Ala Arg
   305 310 315 320
   Gly Ala Leu Ile Leu Arg Gly Pro Asn Glu His Ala Ile Ala Cys Gly
   325 330 335
   Ala Met Ala Asn Trp Gln Leu Tyr Arg Met Pro Phe Leu Gly Val Val
   340 345 350
   Thr Ser Gly Met Met Asp Glu Phe Lys Gly Thr Leu Ala Asn Leu Lys
   355 360 365
   Glu Thr Ala Ala Gln Gly Ile Ile Val Ala Ala Glu Asn Arg Gly Asn
   370 375 380
   Gln Trp Tyr Ser Phe Gln Gly Thr Leu Thr Pro Thr Glu Asp Met Arg
   385 390 395 400
   Glu Val Leu Ile Ala Arg Arg Ile Pro Phe Val Tyr Ile Asp Asp Val
   405 410 415
   Glu Met Ile Gly Ala Gly Leu Thr Glu Ala Phe Arg Leu Tyr His Gln
   420 425 430
   Gly Gln Gly Pro Val Val Ile Leu Ala Thr Gln Asn Val Leu Glu Ser
   435 440 445
   Thr Leu Ser Leu Glu Gly Ala Val Cys Asp Pro Ser Pro Ile Pro Val
   450 455 460
   Leu Ser Ala Asp Asp Pro Leu Pro Met Ser Glu Ser Leu Ala Gln Ala
   465 470 475 480
   Ile Ala Leu Ile Asn Arg Gly Pro Glu Arg Leu Val Trp Gln Leu Gly
   485 490 495
   Pro Val Ser Asp Asp Glu Tyr Ala Leu Ile His Asp Ile Ala Asp Ala
   500 505 510
   Ala Gly Ile Ala Leu Val Asp Ser Leu Ala His Pro Gly Ser Ala Pro
   515 520 525
   Lys Tyr Tyr Gln Gly Arg Arg Asn Pro His Tyr Leu Gly Thr Leu Ala
   530 535 540
   Ile Tyr Gly Tyr Ser Pro Arg Val Tyr Asn Phe Leu His Thr Asn Asp
   545 550 555 560
   Lys Leu Asn Ala Met Ser Glu Gln Ser Leu Phe Met Ile Lys Ser Arg
   565 570 575
   Val Ala Gln Ile Thr Thr Pro Phe Ser Asp Gly Arg Leu Glu Arg Lys
   580 585 590
   Val His Leu Val Gln Leu Thr His Asp Asp Arg His Leu Ser Pro Tyr
   595 600 605
   Ala Asp Leu His Leu His Met Asn Cys Leu Ala Phe Leu Arg Thr Val
   610 615 620
   Lys Ala His Leu Asp Val Asp Pro Ala Leu Arg Glu Arg Arg Arg Ala
   625 630 635 640
   Leu Ile Ala Ala Tyr Leu Asp Ser Pro Ser Asp Val Val Ser Gln Leu
   645 650 655
   Pro Ser Leu Pro Met Ser Ala Asn Tyr Phe Phe Cys Gln Leu Asn Arg
   660 665 670
   Val Ile Glu Glu Leu Ile Glu Thr Glu Gly Phe Asp Phe Thr Gly Val
   675 680 685
   Tyr Asp Val Gly Arg Cys Gly Ile Ser Ala Ala Arg Asn Val Ala Lys
   690 695 700
   Thr Arg Arg Gly Phe Ser Gly Trp Tyr Gly Arg Ala Leu Met Gly Asp
   705 710 715 720
   Ala Leu Leu Ala Thr Gly Tyr Leu Ala Tyr Thr Ser Pro Ser His Val
   725 730 735
   Met Ala Phe Ile Gly Asp Gly Ala Lys Gly Ile Val Pro Asp Ile Leu
   740 745 750
   Pro Ala Phe Ile Asp Asn Ile Leu Thr His Pro Gln Leu Leu Asn Lys
   755 760 765
   Ser Ile Thr Val Phe Tyr Leu Cys Asn Gly Gly Leu Ser Val Ile Asn
   770 775 780
   Thr Tyr Gln Glu Arg Ile Leu Phe Asn Arg Thr Ser Arg Gln Met Arg
   785 790 795 800
   Leu Val Asn Val Glu Gln Pro Asp Val Glu Gln Thr Val Asn Asn Phe
   805 810 815
   His Ile Gln Ser Lys Thr Leu Thr His Phe Asp Glu Asp Val Ile Arg
   820 825 830
   Gln Ala Leu Thr Thr Pro His Arg Leu Asn Leu Phe Ser Val Val Leu
   835 840 845
   Gly His Asn Asn Glu Gly Asp Gly Ile Ser Leu Ala Thr Ala Lys Gly
   850 855 860
   Trp Gln Arg Asp Pro Ser Asp His Asp Ala Leu Gln Glu Arg Lys Ala
   865 870 875 880
   Trp Ala Ala Gln Gln Pro Glu Ser Thr Ser Thr Ala Phe Asp Gln Asp
   885 890 895
   Pro Thr Gln Glu Ala Thr Ser
   900
<210> 2
   <211> 904
   <212> PRT
   <213> Serratia marcescens
<400> 2
   Met Arg Ala Ala Thr Ala Ala Cys Arg Asp Arg Arg Gly Leu Cys Arg
   1               5                   10                  15
   Ala Glu Phe Ala Arg Leu Ala Glu Ala Val Thr Pro Phe Trp Leu His
               20                  25                  30
   Lys Glu Leu Ile Met Thr Thr Leu Thr Gly Gln Ala Arg Leu Thr Asn
           35                  40                  45
   Ser Ala Ala Tyr Glu Gln Val Trp Gln Ala Glu Arg Gln Ala Cys Arg
       50                  55                  60
   Thr Asp Ala Asp Pro Asp Thr Leu Thr Val Gly Val Val Val Val Thr
   65 70 75 80
   Arg Asn Pro Ala Phe Phe Gln Thr Gly Leu Ser Val Leu Asn Asp Ile
   85 90 95
   Arg Asp Tyr Val Phe Asn Arg Val His Ile Gln Ser Glu Met Pro Leu
   100 105 110
   Lys Leu Leu Asp Leu Ala Ala Asp Ser Leu Tyr Leu Ala Ala Arg Glu
   115 120 125
   Lys Ala Leu His Phe Leu Lys Gly Gln Asn Lys Ala Ile Asn Val Arg
   130 135 140
   Ile Ile Gln Cys Ala Ser Leu Ala Glu Ala Thr Gly Lys Ile Ile Tyr
   145 150 155 160
   Thr His Ala Leu Glu Gln Arg Pro Glu Phe His Leu Gly Met Leu Phe
   165 170 175
   Tyr Asp Gln Thr Thr Pro Ala Gly Val Asp Asp Ser Ile Glu Gln Ile
   180 185 190
   Asp Arg Asp Leu Asp Ala Phe Tyr Ser Ala Leu Gln Arg Ser Gly Ile
   195 200 205
   Pro Ala Phe Tyr Thr Thr Phe Ser Thr Val Ala Phe Ile Arg Gln Leu
   210 215 220
   Arg Ser Pro Phe Arg Tyr Leu Pro Gln Gln Tyr Arg Glu Ile Val Arg
   225 230 235 240
   Ser Glu Asp Pro Ala Ile Phe Gln Thr Glu Leu Leu Cys Leu Trp Met
   245 250 255
   Asp Phe Phe Glu Met Asn Tyr Thr Asn Arg Arg Val Lys Pro Ile Gly
   260 265 270
   Ala Leu Ala Leu His Asn Thr Leu Gly Glu Gln Leu Ile Gln Phe Phe
   275 280 285
   Glu Arg Thr Ala Ala Glu Arg Trp Leu Val Ser Tyr Tyr Thr Gly Ser
   290 295 300
   Ile Ile Ser Asn Leu Ile Gly Tyr Leu Asp Arg His Ala Glu Ala Arg
   305 310 315 320
   Gly Ala Leu Ile Leu Arg Gly Pro Asn Glu His Ala Ile Ala Cys Gly
   325 330 335
   Ala Met Ala Asn Trp Gln Leu Tyr Arg Met Pro Phe Leu Gly Val Val
   340 345 350
   Thr Ser Gly Met Met Asp Glu Phe Lys Gly Thr Leu Ala Asn Leu Lys
   355 360 365
   Glu Thr Ala Ala Gln Gly Ile Ile Val Ala Ala Glu Asn Arg Gly Asn
   370 375 380
   Gln Trp Tyr Ser Phe Gln Gly Thr Leu Thr Pro Thr Glu Asp Met Arg
   385 390 395 400
   Glu Val Leu Ile Ala Arg Arg Ile Pro Phe Val Tyr Ile Asp Asp Val
   405 410 415
   Glu Met Ile Gly Ala Gly Leu Thr Glu Ala Phe Arg Leu Tyr His Gln
   420 425 430
   Gly Gln Gly Pro Val Val Ile Leu Ala Thr Gln Asn Val Leu Glu Ser
   435 440 445
   Thr Leu Ser Leu Glu Gly Ala Val Cys Asp Pro Ser Pro Ile Pro Val
   450 455 460
   Leu Ser Ala Asp Asp Pro Leu Pro Met Ser Glu Ser Leu Ala Gln Ala
   465 470 475 480
   Ile Ala Leu Ile Asn Arg Gly Pro Glu Arg Leu Val Trp Gln Leu Gly
   485 490 495
   Pro Val Ser Asp Asp Glu Tyr Ala Leu Ile His Asp Ile Ala Asp Ala
   500 505 510
   Ala Gly Ile Ala Leu Val Asp Ser Leu Ala His Pro Gly Ser Ala Pro
   515 520 525
   Lys Tyr Tyr Gln Gly Arg Arg Asn Pro His Tyr Leu Gly Thr Leu Ala
   530 535 540
   Ile Tyr Gly Tyr Ser Pro Arg Val Tyr Asn Phe Leu His Thr Asn Asp
   545 550 555 560
   Lys Leu Asn Ala Met Ser Glu Gln Ser Leu Phe Met Ile Lys Ser Arg
   565 570 575
   Val Ala Gln Ile Thr Thr Pro Phe Ser Asp Gly Arg Leu Glu Arg Lys
   580 585 590
   Val His Leu Val Gln Leu Thr His Asp Asp Arg His Leu Ser Ala Tyr
   595 600 605
   Ala Asp Leu His Leu His Met Asn Cys Leu Ala Phe Leu Arg Thr Val
   610 615 620
   Lys Ala His Leu Asp Val Asp Pro Ala Leu Arg Glu Arg Arg Arg Ala
   625 630 635 640
   Leu Ile Ala Ala Tyr Leu Asp Ser Pro Ser Asp Val Val Ser Gln Leu
   645 650 655
   Pro Ser Leu Pro Met Ser Ala Asn Tyr Phe Phe Cys Gln Leu Asn Arg
   660 665 670
   Val Ile Glu Glu Leu Ile Glu Thr Glu Gly Phe Asp Phe Thr Gly Val
   675 680 685
   Tyr Asp Val Gly Arg Cys Gly Ile Ser Ala Ala Arg Asn Val Ala Lys
   690 695 700
   Thr Arg Arg Gly Phe Ser Gly Trp Tyr Gly Arg Ala Leu Met Gly Asp
   705 710 715 720
   Ala Leu Leu Ala Thr Gly Tyr Leu Ala Tyr Thr Ser Pro Ser His Val
   725 730 735
   Met Ala Phe Ile Gly Asp Gly Ala Lys Gly Ile Val Pro Asp Ile Leu
   740 745 750
   Pro Ala Phe Ile Asp Asn Ile Leu Thr His Pro Gln Leu Leu Asn Lys
   755 760 765
   Ser Ile Thr Val Phe Tyr Leu Cys Asn Gly Gly Leu Ser Val Ile Asn
   770 775 780
   Thr Tyr Gln Glu Arg Ile Leu Phe Asn Arg Thr Ser Arg Gln Met Arg
   785 790 795 800
   Leu Val Asn Val Glu Gln Pro Asp Val Glu Gln Thr Val Asn Asn Phe
   805 810 815
   His Ile Gln Ser Lys Thr Leu Thr His Phe Asp Glu Asp Val Ile Arg
   820 825 830
   Gln Ala Leu Thr Thr Ser His Arg Leu Asn Leu Phe Ser Val Val Leu
   835 840 845
   Gly His Asn Asn Glu Gly Asp Gly Ile Ser Pro Gly His Arg Gln Arg
   850 855 860
   Leu Ala Ala Leu Ile Arg Ala Asp His Asp Ala Leu Gln Glu Arg Lys
   865 870 875 880
   Ala Trp Ala Ala Gln Gln Pro Glu Ser Thr Ser Thr Ala Phe Asp Gln
   885 890 895
   Asp Pro Thr Gln Glu Ala Thr Ser
   900

## Beschreibung

Die vorliegende Erfindung betrifft die Herstellung von Verbindungen der Formel II (siehe unten), die durch die 1,4-Addition von 2-Oxoalkanoaten an die entsprechenden Ketone erzeugt werden können. Die vorliegende Erfindung betrifft auch die Verwendung des PigD-Proteins zur Katalyse von 1,4-Additionen von 2-Oxoalkanoaten und 2-Oxocarbonsäuren, beispielsweise Pyruvat/ Brenztraubensäure oder 2-Oxo-butyrat/ -buttersäure, an aliphatische, aromatische und heterocyclische α,β-ungesättigte Ketone. Die 1,4-Additionen erfolgen hierbei unter CO₂-Abspaltung. Die Verwendung des PigD-Proteins als Katalysator bei den vorstehend genannten 1,4-Additionen ermöglicht die Synthese von Additionsprodukten mit stereoaktiven bzw. chiralen Zentren, wobei die Additionsprodukte mit einem Enantiomerenüberschuss von mehr als 90 % ee (enantiomeric excess) hergestellt werden können. Die für das Verfahren geeigneten aliphatischen, aromatischen und heterocyclischen α,β-ungesättigten Ketone werden durch Formel I dargestellt (siehe unten).

Die Stetter-Reaktion ist die 1,4-Addition von Aldehyden an α,β-ungesättigte Carbonylverbindungen (Stetter, Angewandte Chemie - International Edition 1976, 15, 639; Stetter et al., Organic Reactions 1991, 40, 407). Mit dem Ziel, die Stetter-Reaktion asymmetrisch durchzuführen, wurden in den vergangenen Jahrzehnten verschiedene chirale Katalysatoren entwickelt (Christmann, Angewandte-Chemie-International Edition 2005, 44, 2632). Die intramolekulare, aber vor allem die intermolekulare asymmetrische Stetter-Reaktion stellt eine große Herausforderung dar. Bisher ist es mit Organokatalysatoren möglich, die intermolekulare 1,4-Addition mit maximal 4 % Ausbeute und einem Enantiomerenüberschuss von nur 39 % durchzuführen, wobei die Katalysatorkonzentration deutlich oberhalb des katalytischen Bereiches liegt. Katalysator und Substrat bilden hierbei stabile Additionsprodukte, eine nach Enders et al. denkbare Erklärung, warum die Aktivität immer gering bleibt (Enders et al., Accounts of Chemical Research 2004, 37, 534).

Eine Alternative zu den bekannten Verfahren zur Durchführung der Stetter-Reaktion könnte die Verwendung von Enzymen als Katalysatoren darstellen. Es wurde beispielsweise postuliert, dass die Reaktion von 2-Octenal mit Pyruvat zu 3-Acetyloctanal in einer Stufe der Biosynthese des Sekundärmetaboliten Prodigiosin im weißen Phenotyp von *Serratia marcescens* durch das PigD-Protein katalysiert wird (Salmond et al., Molecular Microbiology 2005, 56, 971).

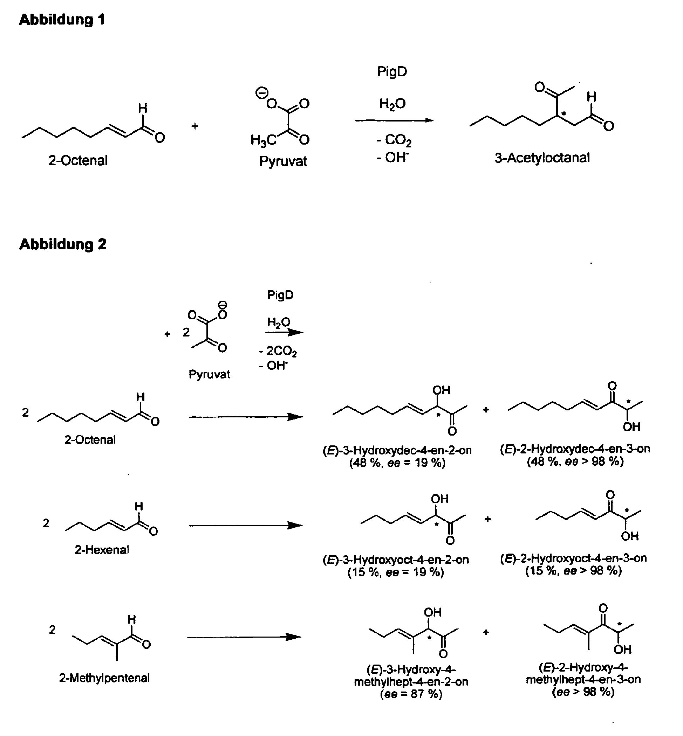

Wie aus der vorgehend genannten Veröffentlichung ersichtlich ist, katalysiert das PigD-Protein vermutlich die Abspaltung von CO₂ aus Pyruvat und die Addition desselben an 2-Octenal in 1,4-Stellung (siehe Abbildung 1).

Über die Stereoselektivität dieser Reaktion ist bislang nichts bekannt. Falls diese 1,4-Aktivität des PigD-Proteins nun gegenüber α,β-ungesättigten Carbonylverbindungen vorhanden wäre, könnte eine intermolekulare Stetter-Reaktion unter Verwendung des PigD-Proteins als Katalysator durchgeführt werden.

Im Rahmen der vorliegenden Erfindung wurde aber überraschenderweise festgestellt, dass die Reaktion von 2-Octenal in der *E*-Konfiguration mit Pyruvat unter PigD-Proteinkatalyse nicht wie von Salmond et al. beschrieben zu einem 1,4-Additionsprodukt, sondern zu einem 1,2-Additionsprodukt führt. Abbildung 2 zeigt die PigD-katalysierte Umsetzung von 2-Octenal und 2-Hexenal mit Pyruvat. Hierbei ist die mittels NMR (Nuclear Magnetic Resonance) bestimmte Produktmenge und der Enantiomerenüberschuss (mittels chiraler HPLC-High Performance Liquid Chromatography bestimmt) in Prozent angegeben. Durch die experimentellen Daten wird daher gezeigt, dass das PigD-Protein eine 1,2-Aktivität aufweist. Die Biosynthese des Sekundärmetaboliten Prodigiosin muss folglich auf einem anderen Reaktionsweg ablaufen.

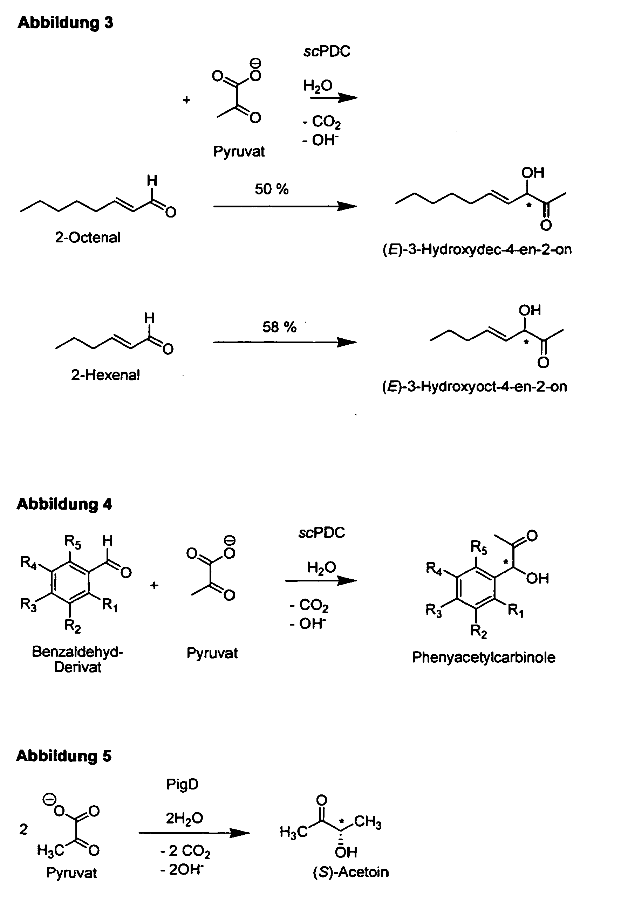

Es ist bekannt, dass Pyruvat Decarboxylasen aus *Saccharomyces cerevisiae, Acetobacter pasteurianus* und *Zymomonas mobilis* C-C-Bindungsknüpfung von aromatischen und aliphatischen Aldehyden in 1,2-Position katalysieren (Bornemann et al., Journal of the Chemical Society-Perkin Transactions 1 1993, 309; M. Pohl, Adv. Biochem. Eng. Biotechnol. 1997, 58, 15; Iding et al., Biochimica et Biophysica Acta-Protein Structure and Molecular Enzymology 1998, 1385, 307; Siegert et al., Protein Engineering Design & Selection 2005, 18, 345). Daher war zu erwarten, dass sie in der Lage sind, ebenso wie das PigD-Protein, eine C-C-Bindungsknüpfung in 1,2-Position bei α,β-ungesättigten aliphatischen Aldehyden zu katalysieren (Abbildung 3).

Weitere Reaktionen zu dem Katalyseverhalten des PigD-Proteins im Rahmen der vorliegenden Anmeldung zeigten, dass auch aromatische Aldehyde mit Pyruvat beispielsweise zu Phenylacetylcarbinolen umgesetzt werden können. Eine derartige Reaktion ist auch für die Pyruvat Decarboxylase aus *Saccharomyces cerevisiae* bekannt (siehe Abbildung 4) (Iding et al., Biochimica et Biophysica Acta-Protein Structure and Molecular Enzymology 1998, 1385, 307). In Abbildung 4 steht "scPDC" für *Saccharomyces cerevisiae* Pyruvat Decarboxylase.

Des Weiteren ist bekannt, dass auch die Pyruvat Decarboxylasen aus *Zymomonas mobilis* und *Acetobacter pasteurianus* die Addition von Pyruvat an Benzaldehyd und 2-Octenal im Sinne einer 1,2-Addition katalysieren.

Des Weiteren katalysiert das PigD-Protein bei Abwesenheit eines Elektrophils, wie beispielsweise einem Aldehyd, die Reaktion von zwei Pyruvat-Molekülen miteinander (siehe Abbildung 5). Diese Reaktivität wurde auch bei verschiedenen Pyruvat Decarboxylasen wie beispielsweise Decarboxylasen aus *Zymomonas mobilis* ((*S*)-Acetoin) oder *Saccharomyces cerevisiae* ((*R*)-Acetoin) festgestellt (Bornemann et al, Journal of the Chemical Society-Perkin Transactions 1 1993, 309).

Das PigD-Protein ist der Sequenzanalyse nach ein Thiamindiphosphat-abhängiges Enzym mit Homologie zu einer Acetolactat Synthase (Marchler-Bauer et al., Nucleic Acid Res 2004, 32, 327).

Aufgrund der vorstehend genannten katalytischen Eigenschaften des PigD-Proteins und den Ähnlichkeiten zu den bekannten Pyruvat Decarboxylasen kann die Schlussfolgerung gezogen werden, dass das PigD-Protein eine Pyruvat Decarboxylase ist.

Die Pyruvat Decarboxylasen sind nicht in der Lage, Additionen an α,β-ungesättigte Ketone zu ermöglichen. Die Pyruvat Decarboxylasen können lediglich Aldehyde als Substrat verwenden. Die fehlende Reaktivität gegenüber Ketonen kann dadurch erklärt werden, dass diese im Vergleich zu Aldehyden eine stark erniedrigte Carbonylaktivität und eine größere sterische Hinderung gegenüber dem aktiven Zentrum der Enzyme aufweisen.

Da die Nacharbeitung der Umsetzung von 2-Octenal mit Pyruvat unerwarteterweise nicht das von Salmond et al. beschriebene 1,4-Additionsprodukt lieferte, war daher zu erwarten, dass das PigD-Protein wie die anderen Decarboxylasen nur zur Katalyse von 1,2-Additionen geeignet ist.

Es war zudem zu erwarten, dass das PigD-Protein, wie auch die anderen Pyruvat Decarboxylasen, nicht in der Lage ist, Additionen an α,β-ungesättigte Ketone zu katalysieren.

Eine Aufgabe der vorliegenden Erfindung ist es daher, eine 1,4-Addition von 2-Oxo-alkanoaten/ -carbonsäuren an α,β-ungesättigte Ketone mit einem hohen Enantiomerenüberschuss und einer guten Ausbeute zu ermöglichen. Hierbei werden Produkte mit *R*-Konfiguration erhalten, wenn R³ ein Aromat (R³ ist über einen sp² hybridisierten Kohlenstoff gebunden) ist. Im Falle eines Aliphaten (R³ ist über einen sp³ hybridisierten Kohlenstoff gebunden) kehrt sich die Konfigurationsbestimmung formal um.

Unerwarteterweise konnte nun festgestellt werden, dass das PigD-Protein entgegen der zu erwartenden Eigenschaften in der Lage ist, 1,4-Additionen von 2-Oxo-alkanoaten/-carbonsäuren an aliphatische, aromatische und heterocyclische α,β-ungesättigte Ketone unter CO₂-Abspaltung zu katalysieren. Die erfindungsgemäße Aufgabe konnte daher überraschenderweise durch die Verwendung des PigD-Proteins als Katalysator gelöst werden.

Die vorliegende Erfindung betrifft daher ein Verfahren zur Herstellung von Verbindungen der Formel II, 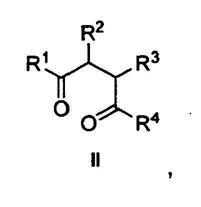 **dadurch gekennzeichnet, dass** Verbindungen der Formel I, 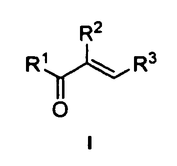
mit 2-Oxo-alkanoaten/ -carbonsäuren der Formel R⁴C(O)COO⁻/ R⁴C(O)COOH umgesetzt werden,
worin R¹ einen geradkettigen oder verzweigten, bevorzugt geradkettigen C₁-C₁₀-Alkylrest oder einen Aromaten, wobei der Aromat weiter bevorzugt eine Phenylgruppe darstellt,
R² einen Wasserstoff, einen Alkylrest, bevorzugt einen C₁-C₁₀-Alkylrest, oder einen Aromaten, wobei der Aromat weiter bevorzugt eine Phenylgruppe darstellt,
R³ einen geradkettigen oder verzweigten C₁₋₁₀-Alkylrest, einen Heterocyclus oder einen Aromaten, wobei der C₁₋₁₀-Alkylrest wiederum einen Heterocyclus oder einen Aromaten aufweisen kann, und wobei der Heterocyclus oder der Aromat mit Methoxygruppen, Halogenen oder Hydroxygruppen substituiert sein können, wobei der Aromat weiter bevorzugt eine Phenylgruppe darstellt, und
R⁴ einen Alkylrest, bevorzugt C₁₋₁₀-Alkylrest, darstellt,
und worin das PigD-Protein als Katalysator verwendet wird. Es ist zudem weiter bevorzugt, dass R¹ einen gesättigten Alkylrest darstellt. Noch weiter bevorzugt werden die Verbindungen der Formel I in der *E*-Konfiguration eingesetzt.

In einer noch weiter bevorzugten Ausführungsform des Verfahrens stellt R¹ eine Methylgruppe oder eine Phenylgruppe, R² einen Wasserstoff, eine Alkylgruppe, bevorzugt einen C₁₋₄-Alkylrest, weiter bevorzugt eine Methylgruppe, oder eine Phenylgruppe, R³ eine C₁₋₁₀-Alkylgruppe, einen Heterocyclus oder einen Aromaten, die mit Methoxygruppen, Halogenen oder Hydroxygruppen substituiert sein können, und R⁴ einen C₁-C₁₀-Kohlenwasserstoff dar. Hierbei stellt der Aromat weiter bevorzugt eine Phenylgruppe dar. Bevorzugterweise ist die C₁₋₁₀-Alkylgruppe eine gesättigte Alkylgruppe.

In einer anderen bevorzugten Ausführungsform des Verfahrens erfolgt die 1,4-Addition enantioselektiv mit einem Enantiomerenüberschuss von mehr als 80 % ee. Weiter bevorzugt erfolgt die 1,4-Addition enantioselektiv mit einem Enantiomerenüberschuss von mehr als 90 % ee und am meisten bevorzugt mit einem Enantiomerenüberschuss von mehr als 98 % ee.

In einer weiter bevorzugten Ausführungsform des Verfahrens werden geradkettige, gesättigte 2-Oxo-alkanoate/ -carbonsäuren mit 1-10 Kohlenstoffatomen als Donorsubstrate verwendet.

In einer anderen bevorzugten Ausführungsform des Verfahrens wird Pyruvat oder Brenztraubensäure oder 2-Oxobutanoat oder 2-Oxobuttersäure als Donorsubstrat verwendet und R⁴ ist gleich Methyl oder Ethyl. Weiter bevorzugt wird Pyruvat oder Brenztraubensäure als Donorsubstrat verwendet.

Die vorliegende Erfindung betrifft auch die Verwendung des PigD-Proteins zur Katalyse von 1,4-Additionen von 2-Oxoalkanoaten oder 2-Oxocarbonsäuren an α,β-ungesättigte Ketone der Formel I 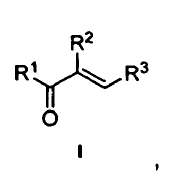
worin R¹ einen geradkettigen oder verzweigten C₁-C₁₀-Alkylrest oder einen Aromaten,
R² einen Wasserstoff oder Aromaten, wobei der Aromat bevorzugt eine Phenylgruppe darstellt, und
R³ einen geradkettigen oder verzweigten C₁₋₁₀-Alkylrest, einen Heterocyclus oder einen Aromaten darstellt, wobei die C₁₋₁₀-Alkylgruppe wiederum einen Heterocyclus oder einen Aromaten aufweisen kann, und wobei der Heterocyclus oder der Aromat mit Methoxygruppen, Halogenen oder Hydroxygruppen substituiert sein kann, und
wobei die 1,4-Additionen unter CO₂-Abspaltung erfolgen. Weiter bevorzugt werden die Verbindungen der Formel I in der *E*-Konfiguration verwendet.

Die Reste R¹, R² und R³ können bei dem erfindungsgemäßen Herstellungsverfahren und bei dem Verfahren zur Verwendung des PigD-Proteins gleichermaßen definiert werden.

Eine bevorzugte Ausführungsform der Verwendung des PigD-Proteins ist **dadurch gekennzeichnet, dass** Pyruvat/ Brenztraubensäure oder 2-Oxo-butanoat/ -buttersäure als 2-Oxoalkanoat verwendet wird.

Eine weiter bevorzugte Ausführungsform der Verwendung des PigD-Proteins ist **dadurch gekennzeichnet, dass** die 1,4-Additionen enantioselektiv mit einem Enantiomerenüberschuss der Additionsprodukte von mehr als 80% ee, weiter bevorzugt von mehr als 90 % ee und am meisten bevorzugt mit einem Enantiomerenüberschuss der Additionsprodukte von mehr als 98 % ee erfolgen.

Eine andere bevorzugte Ausführungsform der Verwendung des PigD-Proteins ist **dadurch gekennzeichnet, dass** aliphatische, aromatische und heterocyclische α,β-ungesättigte Ketone gemäß der Formel I eingesetzt werden, worin
R¹ eine Methylgruppe oder einen Aromaten, wobei der Aromat bevorzugt eine Phenylgruppe darstellt, R² ein Wasserstoffatom, eine Alkylgruppe oder Aromaten, weiter bevorzugt ein Wasserstoffatom oder Aromaten, und R³ eine C₁₋₁₀-Alkylgruppe, einen Heterocyclus oder eine Phenylgruppe, wobei der Heterocyclus und die Phenylgruppe mit Methoxygruppen, Halogenen oder Hydroxygruppen substituiert sein kann, darstellt.

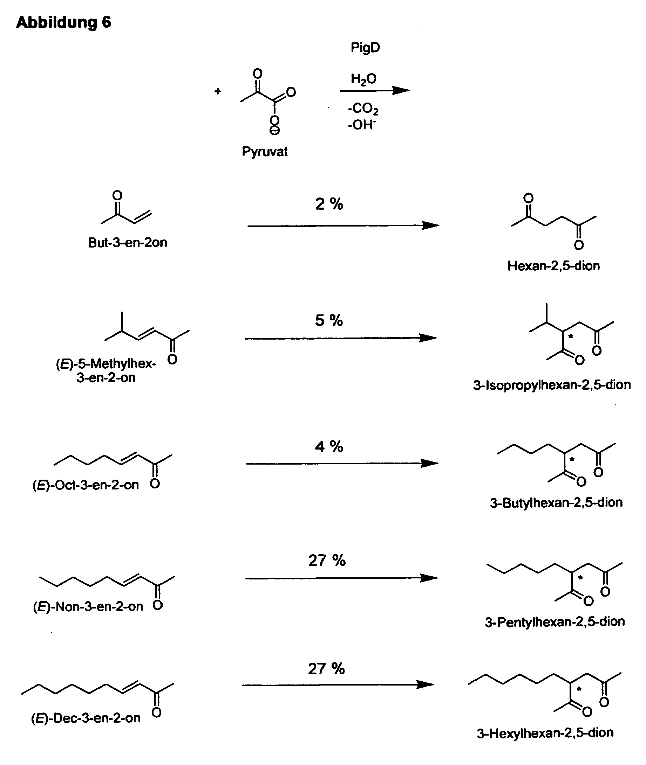

Überraschenderweise ist es mit der erfindungsgemäßen Reaktion möglich, eine 1,4-Addition von 2-Oxoalkanoaten oder 2-Oxocarbonsäuren an aliphatische geradkettige oder verzweigte α,β-ungesättigte Ketone (siehe Abbildung 6) mit einem sehr hohen Enantiomerenüberschuss (siehe Beispiele) durchzuführen. Zudem bleibt die Menge an Nebenprodukten (aus der Reaktion der 2-Oxo-alkanoate/ -carbonsäuren untereinander) bei der Verwendung des PigD-Proteins gering.

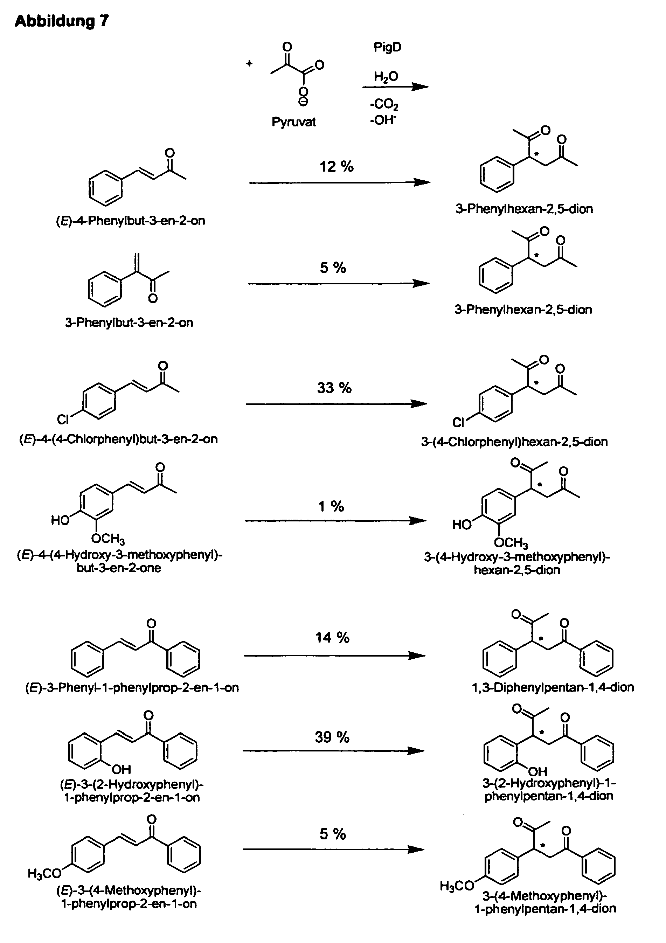

Des Weiteren ist es mit der erfindungsgemäßen Reaktion möglich, eine 1,4-Addition von 2-Oxo-alkanoaten/ -carbonsäuren an aromatische α,β-ungesättigte Ketone (siehe Abbildung 7) mit einem hohen Enantiomerenüberschuss durchzuführen

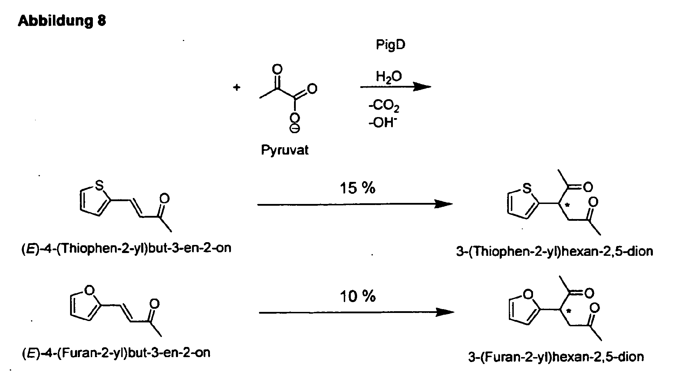

Ebenso können auf vorteilhafte Weise, ausgehend von α,β-ungesättigten heterocyclischen Ketonen, unter Verwendung des PigD-Proteins, 1,4-Diketone hergestellt werden (siehe Abbildung 8).

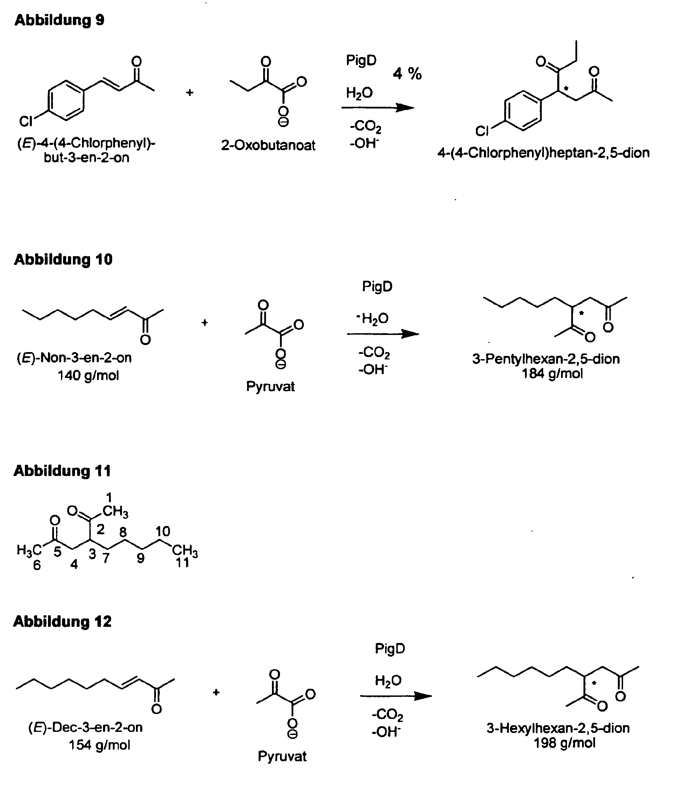

Auf vorteilhafte Weise können zudem auch andere 2-Oxo-alkanoate/ -carbonsäuren, wie beispielsweise 2-Oxo-butanoat/ -buttersäure, eingesetzt werden (siehe Abbildung 9).

Das PigD-Protein katalysiert 1,4-Additionen von 2-Oxo-alkanoaten/ -carbonsäuren an aliphatische, aromatische und heterocyclische α,β-ungesättigte Ketone unter CO₂-Abspaltung (siehe Gleichung 1; Gleichung 1 zeigt nur die Verwendung von 2-Oxo-alkanoaten, 2-Oxo-carbonsäuren können ebenfalls verwendet werden). Der Rest R⁴ in der allgemeinen Formel für die 2-Oxo-alkanoate/ -carbonsäure steht für geradkettige oder verzweigte, bevorzugt geradkettige Alkylreste, bevorzugt geradkettige oder verzweigte, bevorzugt geradkettige (z.B. Methyl-, Ethyl-, Propyl-, Butyl-, Pentyl- usw.) C₁-C₁₀-Alkylreste.

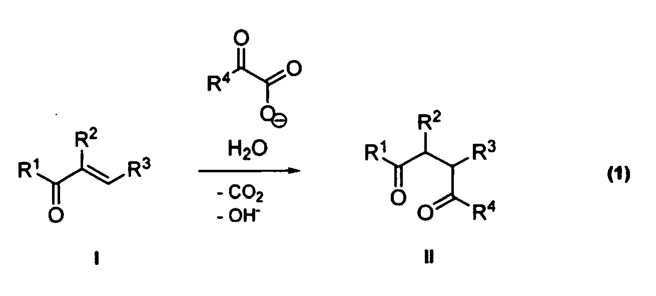

Mit der erfindungsgemäßen Reaktion können 2-Oxo-alkanoate/ -carbonsäuren mit Verbindungen der Formel I zu Verbindungen der Formel II umgesetzt werden. Die Beschreibung der Reaktion bezieht sich daher immer sowohl auf die Verwendung des PigD-Proteins zur Katalyse der erfindungsgemäßen 1,4-Additionen, als auch auf die Herstellung von Verbindungen der Formel II unter Verwendung der erfindungsgemäßen 1,4-Additionen.

Das PigD-Protein katalysiert die erfindungsgemäße Reaktion stereoselektiv. Bevorzugt wird das neu gebildete stereoaktive bzw. chirale Zentrum mit einem Enantiomerenüberschuss von mindestens 80 % ee, weiter bevorzugt mindestens 86 % ee, weiter bevorzugt mindestens 90 % ee, weiter bevorzugt mindestens 94 % ee, weiter bevorzugt mindestens 98 % ee gebildet.

Der Ausdruck "PigD-Protein", so wie hier verwendet, umfasst Proteine, die im Wesentlichen die Aminosäuresequenzen gemäß SEQ ID NO: 1 (modifiziertes PigD-Protein, siehe Beispiele) oder SEQ ID NO: 2 (bekanntes PigD-Protein, EMBL Datenbanknummer: CAH55649.1) aufweisen (siehe Sequenzprotokoll). Besonders bevorzugt weist das PigD-Protein im Wesentlichen die Aminosäuresequenz gemäß SEQ ID NO: 1 auf.

Der Ausdruck "PigD-Protein" umfasst ebenfalls Proteinvarianten der Sequenz gemäß SEQ ID NO: 1, beispielsweise aufgrund von Allel-Variationen. Diese Varianten können Mutationen bezüglich der Sequenz gemäß SEQ ID NO: 1 aufweisen, beispielsweise Substitutionen, Additionen und/oder Deletionen. Hierbei können bevorzugt 1-30, weiter bevorzugt 1-25, weiter bevorzugt 1-20, weiter bevorzugt 1-15, weiter bevorzugt 1-10, weiter bevorzugt 1-5 und noch weiter bevorzugt 1-3 Aminosäuren substituiert, deletiert und/ oder addiert sein, relativ zu der Sequenz gemäß SEQ ID NO: 1. Die katalytische Aktivität muss dabei aber mindestens beibehalten werden. Bevorzugt ist eine Erhöhung der katalytischen Aktivität durch geeignete Mutation oder Mutationen.

Der Ausdruck "Aromat", so wie hier verwendet, umfasst substituierte und unsubstituierte aromatische Kohlenwasserstoffverbindungen, bevorzugt unsubstituierte Kohlenwasserstoffverbindungen. Weiter bevorzugt ist der Aromat eine substituierte oder unsubstituierte Phenylgruppe. Der Ausdruck "Phenylgruppe", so wie hier verwendet umfasst substituierte und unsubstituierte Phenylgruppen, bevorzugt eine unsubstituierte Phenylgruppe. Der Ausdruck "substituiert", so wie hier verwendet, umfasst bevorzugt die Substitution mit Methoxygruppen, Halogenen oder Hydroxygruppen, weiter bevorzugt die Substitution mit 1-3, bevorzugt mit einer Methoxygruppe, mit 1-3, bevorzugt mit einem Halogen oder mit 1-3, bevorzugt mit einer Hydroxygruppe. Der Ausdruck "Heterocyclus", so wie hier verwendet umfasst bevorzugt Heterocyclen mit 1-3, weiter bevorzugt 1-2, am meisten bevorzugt ein Atom(e) aus der folgenden Gruppe auf: Sauerstoff, Schwefel, Stickstoff, Selen. Weiter bevorzugt ist der Heterocyclus ein 5-Ring oder ein 6-Ring.

Bei der erfindungsgemäßen Reaktion werden 2-Oxo-alkanoate/ -carbonsäuren als Donorsubstrate verwendet, die durch eine enzymkatalysierte nicht-oxidative Decarboxylierung eine "Umpolungsreaktion" erfahren. Das hierbei entstehende nucleophile Carbanion kann dann in einer 1,4-Addition im Sinne einer Stetter-Reaktion an den β-Kohlenstoff des Ketons addieren.

Die hierbei geeigneten 2-Oxo-alkanoate/ -carbonsäuren umfassen geradkettige oder verzweigte, bevorzugt geradkettige gesättigte 2-Oxo-alkanoate/ -carbonsäuren, weiter bevorzugt besitzen die 2-Oxo-alkanoate/ -carbonsäuren 3-12, weiter bevorzugt 3-8, weiter bevorzugt 3-6, weiter bevorzugt 3-5, weiter bevorzugt 3 oder 4 und am meisten bevorzugt 3 C-Atome. Bevorzugt entspricht dies der Formel R⁴C(O)COO⁻/ R⁴C(O)COOH mit einem Rest R⁴ mit 1-10 C-Atomen, weiter bevorzugt 1-6 C-Atomen, weiter bevorzugt 1-4 C-Atomen, noch weiter bevorzugt 1-3 C-Atomen, weiter bevorzugt 1 oder 2 C-Atomen und am meisten bevorzugt einem 1 C-Atom.

Die für die Reaktion geeigneten Akzeptorsubstrate bzw. Ketone umfassen aliphatische, aromatische und heterocyclische α,β-ungesättigte Ketone. Diese Ketone werden durch Formel I dargestellt.

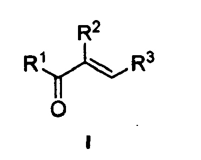

### In Formel I sind folgende Reste bevorzugt:

R¹ kann einen geradkettigen oder verzweigten, bevorzugt geradkettigen C₁-C₁₀-Alkylrest oder Aromaten darstellen, wobei der Aromat weiter bevorzugt eine Phenylgruppe darstellt welche auch substituiert sein kann;
R² kann einen Wasserstoff, einen C₁₋₁₀-Alkylrest oder Aromaten darstellen, weiter bevorzugt einen Wasserstoff, oder einen Aromaten, wobei der Aromat weiter bevorzugt eine Phenylgruppe darstellt;
R³ kann einen geradkettigen oder verzweigten C₁₋₁₀-Alkylrest, einen Heterocyclus oder einen Aromaten darstellen, wobei der C₁₋₁₀-Alkylrest wiederum einen Heterocyclus oder einen Aromaten aufweisen kann, und wobei der Heterocyclus oder der Aromat mit Methoxygruppen, Halogenen oder Hydroxygruppen substituiert sein kann;
R⁴ kann einen geradkettigen oder verzweigten C₁-C₁₀-Alkylrest darstellen.

### In Formel I sind folgende Reste noch weiter bevorzugt:

R¹ kann eine Methylgruppe oder Phenylgruppe darstellen;
R² kann einen Wasserstoff oder Aromaten darstellen, wobei der Aromat weiter bevorzugt eine Phenylgruppe darstellt;
R³ kann eine C₁₋₁₀-Alkylgruppe, einen Heterocyclus oder eine Phenylgruppe darstellen, die mit Methoxygruppen, Halogenen oder Hydroxygruppen substituiert sein kann, und R⁴ einen C₁-C₁₀-Kohlenwasserstoff dar. Bevorzugterweise ist die C₁₋₁₀-Alkylgruppe hierbei eine gesättigte Alkylgruppe.

### In Formel I sind folgende Reste noch weiter bevorzugt:

R¹ ist eine Methylgruppe oder Phenylgruppe.
R² ist ein Wasserstoffatom oder Aromat, wobei der Aromat weiter bevorzugt eine Phenylgruppe darstellt,
R³ ist eine C₁₋₁₀-Alkylgruppe, ein Heterocyclus oder eine Phenylgruppe, wobei der Heterocyclus oder die Phenylgruppe mit 1-3, bevorzugt mit einer Methoxygruppe, mit 1-3, bevorzugt mit einem Halogen oder mit 1-3, bevorzugt mit einer Hydroxygruppe substituiert sein kann. Bevorzugt weist der Heterocyclus hierbei 1-3, weiter bevorzugt 1-2, am meisten bevorzugt ein Atom(e) aus der folgenden Gruppe auf: Sauerstoff, Schwefel, Stickstoff, Selen. Weiter bevorzugt ist der Heterocyclus ein 5-Ring oder ein 6-Ring.

Mit der erfindungsgemäßen Reaktion können aus 2-Oxo-alkanoaten/ -carbonsäuren und den Verbindungen der Formel I unter Verwendung des PigD-Proteins Verbindungen gemäß der Formel II hergestellt werden.

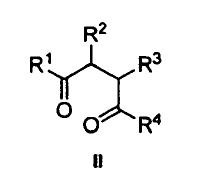

### In Formel II sind folgende Reste bevorzugt:

Die Reste R¹ bis R³ entsprechen den Resten wie für Formel I definiert.

R⁴ ist bevorzugt ein geradkettiger oder verzweigter, bevorzugt ein geradkettiger Kohlenwasserstoff mit 1-10 C-Atomen, weiter bevorzugt 1-6 C-Atomen, weiter bevorzugt 1-4 C-Atomen, noch weiter bevorzugt 1-3 C-Atomen, weiter bevorzugt 1 oder 2 C-Atomen und am meisten bevorzugt einem C-Atom. Weiter bevorzugt ist R⁴ ein gesättigter Kohlenwasserstoff.

Die erfindungsgemäße -1,4-Addition wird zusätzlich bevorzugt mit einem Stoffmengenverhältnis von Donorsubstrat (2-Oxo-alkanoat/ -carbonsäure):Akzeptorsubstrat (Ketonverbindung) von 3:1 bis 1:3, weiter bevorzugt 2:1 bis 1:2, weiter bevorzugt von 1,5:1 bis 1:2 und am meisten bevorzugt von 1,5:1 bis 1:1, durchgeführt.

Die 1,4-Addition kann unter der Verwendung von Lösungsmitteln oder Lösungsmittelgemischen erfolgen die dem Fachmann bekannt sind. Bevorzugterweise wird eine Mischung aus Methyltertiärbutylether oder Dimethylsulfoxid und Wasser verwendet (siehe Beispiel 1). Die Herstellung des PigD-Proteins ist in den Beispielen beschrieben und mit Techniken möglich, die dem Fachmann bekannt sind. Die Durchführung der Reaktionen ist ebenfalls in den Beispielen beschrieben. Die Größe der Reaktionsansätze ist nicht beschränkt.

Die 1,4-Addition kann bevorzugt unter Schütteln oder Rühren bei beispielsweise 30 °C durchgeführt werden. Zusätzlich ist es bevorzugt, dass zunächst nur ein Teil des PigD-Proteins in den Reaktionsansatz gegeben wird und nach einer Zeit von mehreren Stunden, bevorzugt nach 10-25 Stunden, die Restmenge an PigD-Protein zugegeben wird. Das Reaktionsende kann mit üblichen Verfahren wie beispielsweise GC-MS (Gaschromatographie mit Massenspektrometrie gekoppelt) bestimmt werden. Bevorzugterweise wird das Reaktionsgemisch nach 10-45 Stunden, weiter bevorzugt nach 20-45 Stunden aufgearbeitet.

Die Aufarbeitung des Reaktionsgemisches kann nach bekannten Verfahren erfolgen. Hierbei wird zunächst das Protein beispielsweise durch Abnutschen über ein Cellite-Pad entfernt und das Rohprodukt durch Nachwaschen mit einem organischen Lösungsmittel, beispielsweise Ethylacetat, in die organische Phase überführt. Die organische Phase kann anschließend beispielsweise über Natriumsulfat getrocknet und das Lösungsmittel im Vakuum abgedampft werden. Eine Reinigung des Rohprodukts kann mittels Säulenchromatographie erfolgen (siehe Beispiel 1).

### Beschreibung der Abbildungen

**Abbildung 1** zeigt die von Salmond et al. postulierte Reaktion von 2-Octenal mit Pyruvat unter PigD-Katalyse.

**Abbildung 2** zeigt die PigD-katalysierte Umsetzung von 2-Octenal, 2-Hexenal und 2-Methylpentenal mit Pyruvat. In Klammern ist die Produktbildung in Prozent (mittels NMR-Nuclear Magnetic Resonance- bestimmt) und der Enantiomerenüberschuss (mittels chiraler HPLC-High Performance Liquid Chromatography bestimmt) angegeben.

**Abbildung 3** zeigt die Addition von Pyruvat an α,β-ungesättigte aliphatische Aldehyde unter Katalyse mit einer Pyruvat Decarboxylase, beispielsweise aus *Saccharomyces cerevisiae.* Die Prozentangaben der Ausbeuten für die Pyruvat Decarboxylase aus *Saccharomyces cerevisiae* sind in der Abbildung auf den Reaktionspfeilen dargestellt.

**Abbildung 4** zeigt *sc*PDC katalysierte Umsetzungen mit Benzaldehyd und Benzaldehyd-Derivaten. In Abbildung 4 steht "*sc*PDC" für *Saccharomyces cerevisiae* Pyruvat Decarboxylase. Reaktionsbedingungen: 1.5 mL Reaktionsvolumen, 10 % Ethanol, 20 mM Benzaldehyd-Derivat, 50 mM Natrium Pyruvat, 30 µL *sc*PDC zellfreies Rohextrakt (370 U/mL).

**Abbildung 5** zeigt die PigD-katalysierte Umsetzung von Pyruvat zu Acetoin (*ee* (S)-Acetoin = 70 %, chirale GC (FS-Lipodex D, 70 °C, Rₜ = 13.7 min (*R*), 18.3 (*S*)).

**Abbildung 6** zeigt die Verwendung von α,β-ungesättigten aliphatischen Ketonen als Akzeptorsubstrat. Die Produktbildung ist in Prozent (mittels NMR bestimmt) angegeben. Der Reaktionsansatz setzte sich bei diesen Reaktionen zusammen aus: 1.5 mL Reaktionsvolumen, 20 % DMSO (Dimethylsulfoxid), 20 mM Akzeptorsubstrat, 25 mM Natrium Pyruvat, 1100 µg PigD-Protein.

**Abbildung 7** zeigt die Verwendung von α,β-ungesättigten aromatischen Ketonen als Akzeptorsubstrat. Die Produktbildung ist in Prozent (mittels NMR bestimmt) angegeben. Der Reaktionsansatz setzte sich bei diesen Reaktionen zusammen aus: 1.5 mL Reaktionsvolumen, 20 % DMSO, 20 mM Akzeptorsubstrat, 25 mM Natrium Pyruvat, 1100 µg PigD Protein.

**Abbildung 8** zeigt die 1,4-Addition von Pyruvat an α,β-ungesättigte heterocyclische Ketone. Die Produktbildung, mittels NMR bestimmt, ist hierbei auf dem Reaktionspfeil in % angegeben. Der Reaktionsansatz setzte sich bei diesen Reaktionen zusammen aus: 1.5 mL Reaktionsvolumen, 20 % DMSO, 20 mM Akzeptor Substrat, 25 mM Natrium Pyruvat, 1100 µg PigD Protein.

**Abbildung 9** zeigt die Verwendung von 2-Oxobutanoat als Donorsubstrat. Die Produktbildung, mittels NMR bestimmt, ist hierbei auf dem Reaktionspfeil in % angegeben. Der Reaktionsansatz war bei dieser Reaktion: 1.5 mL Reaktionsvolumen, 20 % DMSO, 20 mM Akzeptor Substrat, 25 mM 2-Oxobutanoat, 1100 µg PigD Protein.

**Abbildung 10** zeigt die PigD-katalysierte Umsetzung von (*E*)-Non-3-en-2-on zu 3-Pentylhexan-2,5-dion.

**Abbildung 11** zeigt das Nummerierungsschema von 3-Pentylhexan-2,5-dion.

**Abbildung 12** zeigt die PigD-katalysierte Umsetzung von (*E*)-Dec-3-en-2-on zu 3-Hexylhexan-2,5-dion.

**Abbildung 13** zeigt die PigD-katalysierte Umsetzung von (*E*)-4-Phenylbut-3-en-2-on zu 3-Phenylhexan-2,5-dion.

**Abbildung 14** zeigt die PigD-katalysierte Umsetzung von (*E*)-4-(4-Chlorphenyl)but-3-en-2-on zu 3-(4-Chlorphenyl)hexan-2,5-dion.

**Abbildung 15** zeigt die PigD-katalysierte Umsetzung von (*E*)-4-(Furan-2-yl)but-3-en-2-on zu 3-(Furan-2-yl)hexan-2,5-dion.

**Abbildung 16** zeigt die Enantiomerenüberschüsse der durch die PigD-Katalyse entstandenen Produkte.
Anmerkung "a" in Abbildung 16: Da über verschiedene Analysenmethoden nur ein Peak detektiert wurde, wurde schlussgefolgert, dass hier der Enantiomerenüberschuss des enzymatisch gebildeten Produktes bei ee > 99 % liegt.

**Abbildung 17** zeigt die chemische Synthese des racemischen 3-Pentylhexan-2,5-dion. Die Abkürzung MMPP steht für Magnesium Monoperoxyphthalat, DBU für 1,8-Diazabicyclo[5.4.0]undec-7-en.

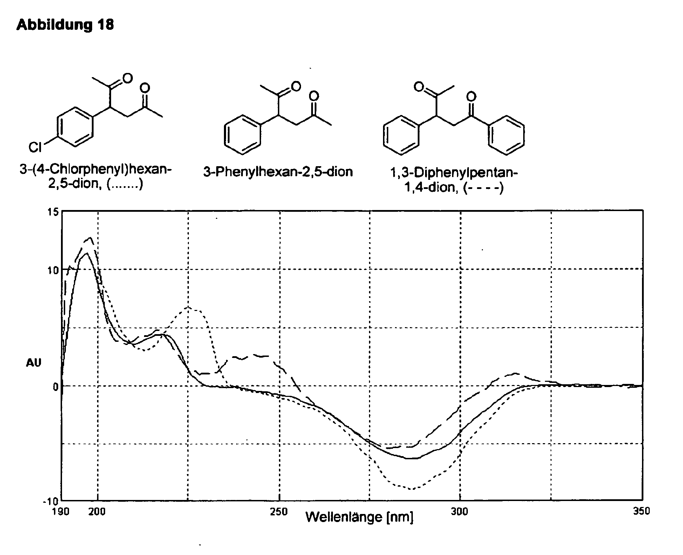

**Abbildung 18** zeigt die CD Spektren von drei Produkten, die durch PigD-Katalyse hergestellt wurden.

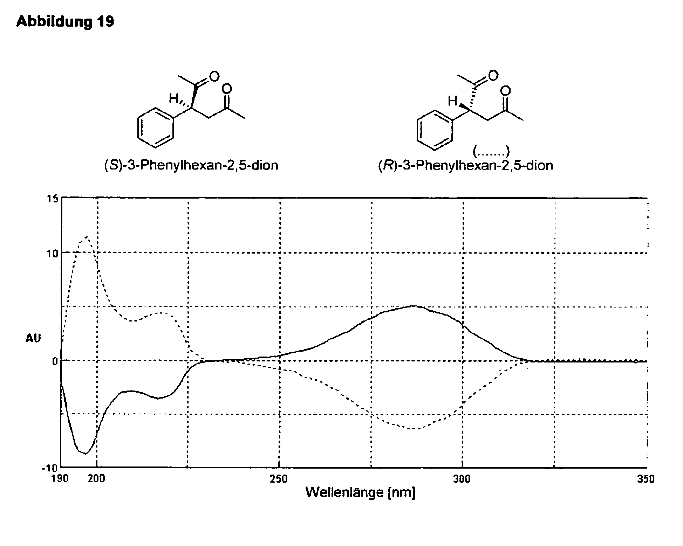

**Abbildung 19** zeigt das CD Spektrum von (S)-3-Phenylhexan-2,5-dion (durch chemische Synthese hergestellt) und von (*R*)-3-Phenylhexan-2,5-dion (durch PigD-Katalyse hergestellt).

**Abbildung 20** zeigt den pET22b(+)-Vektor (Novagen).

**Abbildung 21** zeigt die Klonierungsstellen des pET22b(+)-Vektors (Novagen).

**Abbildung 22** zeigt den Klonierungsvektor (Novagen), in den das aus *Serratia marcescens* gewonnene Gen pigD insertiert wurde.

### Beispiel 1: Reaktionsbedingungen und Aufarbeitung

Die Reaktionen erfolgten im 12 mL Maßstab, können aber in jeglichen Ansatzgrößen durchgeführt werden.

Es wurden jeweils 20 mM Akzeptorsubstrat und 25 mM Natrium Pyruvat eingesetzt. Die Substrate wurden in 600 µL Methyltertiärbutylether gelöst und 11.4 mL demineralisiertes Wasser zugegeben. Nach Zugabe des lyophilisierten Proteins PigD wurde bei 30 °C und 300 rpm geschüttelt.

In den Ansätzen zu den Abbildungen 10, 12 und 13 wurden zu Beginn 50 % des angegebenen Proteins und nach 20 Stunden die weiteren 50 % zugegeben. Das Reaktionsgemisch wurde nach 40 Stunden aufgearbeitet.

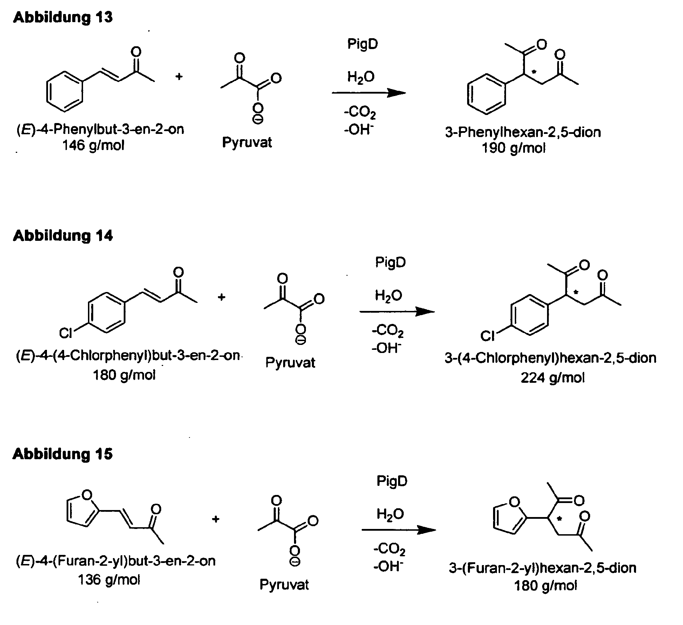

In den Ansätzen der Abbildungen 14 und 15 erfolgte die gesamte Proteinzugabe zum Zeitpunkt Null und es wurde nach 20 Stunden aufgearbeitet.

Die Proteinlyophilisate enthielten 10 % PigD-Protein. Unten angegeben sind die tatsächlich im Ansatz enthaltenen Proteinendkonzentrationen.

Die Entsalzung bei der Proteinreinigung wurde mit folgendem Puffer durchgeführt: 10 mM KPᵢ, 2.5 mM MgSO₄, 0.1 mM ThDP, 0.005 mM FAD, pH 7.0. Somit lässt sich die Pufferkonzentration der Ansätze wie folgt berechnen:

Abbildung 10:
Stunde 1-20: 30 mM KPᵢ, 7.5 mM MgSO₄, 0.3 mM ThDP, 0.015 mM FAD
Stunde 20-40: 60 mM KPᵢ, 15 mM MgSO₄, 0.6 mM ThDP, 0.03 mM FAD

Abbildung 12:
Stunde 1-20: 43 mM KPᵢ, 11 mM MgSO₄, 0.4 mM ThDP, 0.02 mM FAD
Stunde 20-40: 46 mM KPᵢ, 22 mM MgSO₄, 0.8 mM ThDP, 0.04 mM FAD

Abbildung 13:
Stunde 1-20: 70 mM KPᵢ, 17.5 mM MgSO_{4,} 0.7 mM ThDP, 0.035 mM FAD
Stunde 20-40: 140 mM KPᵢ, 35 mM MgSO₄, 1.4 mM ThDP, 0.07 mM FAD

Abbildung 14:
46 mM KPᵢ, 11.5 mM MgSO₄, 0.5 mM ThDP, 0.023 mM FAD

Abbildung 15:
83 mM KPᵢ, 20.75 mM MgSO₄, 0.83 mM ThDP, 0.04 mM FAD

Aufarbeitung:
Das Protein wurde durch Abnutschen über ein Cellite-Pad entfernt und das Rohprodukt durch Nachwaschen mit 60 mL Ethylacetat in die organische Phase überführt. Die organische Phase wurde über Na₂SO₄ getrocknet und das Lösungsmittel im Vakuum eingeengt. Das Rohprodukt wurde durch Säulenchromatographie gereinigt (SiO₂, Cyclohexan/ Ethylacetat = 5:1).

### Beispiel 2: PigD-katalysierte Umsetzung von (E)-Non-3-en-2-on zu 3-Pentylhexan-2,5-dion

Die PigD-katalysierte Umsetzung von (*E*)-Non-3-en-2-on zu 3-Pentylhexan-2,5-dion ist in Abbildung 10 dargestellt. Reaktionsansatz und Analyse des Produkts (siehe Abbildung 11): 14 mg Protein; 48 % Ausbeute an 3-Pentylhexan-2,5-dion laut NMR Messung des Rohproduktes; Nach Reinigung über Säulenchromatographie (R_{f}(Cyc)ohexan/ Ethylacetat = 5:1) = 0.34): 17 mg 3-Pentylhexan-2,5-dion (38 % Ausbeute);

GCMS Rₜ = 8.49 min. MS (70 eV, El); *m*/*z*: 184 ([M⁺], 1 %), 169 ([M⁺ - CH₃], 3 %), 141 ([M⁺ - C₂H₃O], 10 %), 127 ([M⁺ - C₃H₅O], 25 %), 114 ([C₆H₁₀O₂⁺), 80 %), 71 ([C₅H₁₁⁺], 100 %).

¹H NMR (400 MHz, CDCl₃): δ = 3.07-2.98 (m, 1 H, 3), 2.97 (dd, *J* = 17.3 Hz, *J* = 9.9 Hz, 1 H, 4), 2.44 (dd, *J* = 17.3 Hz, *J* = 3.0 Hz, 1 H, 4), 2.24 (s, 3 H, 1), 2.15 (s, 3 H, 6), 1.52-1.67 (m, 2 H, 7), 1.20-1.35 (m, 6 H, 8, 9, 10), 0.89 (t, 3 H, 11).

¹³C NMR (77.2 MHz, CDCl₃): δ = 211.6, 207.5, 46.8 (4), 44.7 (3), 31.7 (8), 31.2 (7), 29.9 (6), 29.7 (1), 26.7 (9), 22.4 (10), 13.9 (11).

Chirale GC (FS-Lipodex D, isokratisch, 90 °C) Rₜ = 98.9 min (Hauptenantiomer), 102.6 min (*ent*). *ee* = 99.4 % (nach Säulenchromatographie); ee > 99.4 % (direkt extrahiert nach 40 Stunden bei 30 °C). Der Enantiomerenüberschuss wurde über Gaschromathographie an chiraler Phase mit dem chemisch synthetisierten Racemat als Referenz ermittelt.

### Beispiel 3: PigD-katalysierte Umsetzung von (E)-Dec-3-en-2-on zu 3-Hexylhexan-2,5-dion

Die PigD katalysierte Umsetzung von (*E*)-Dec-3-en-2-on zu 3-Hexylhexan-2,5-dion ist in Abbildung 12 dargestellt. Reaktionsansatz und Analyse des Produkts: 20.4 mg Protein; 30 % 3-Hexylhexan-2,5-dion im Rohprodukt (GCMS); Nach Reinigung über Säulenchromatographie (R_{f}(Cyc)ohexan/ Ethylacetat = 5:1) = 0.34): 14.5 mg 3-Hexylhexan-2,5-dion (30 % Ausbeute).

GCMS Rₜ = 8.49 min. MS (70 eV, EI); *m*/*z*: 198 ([M⁺], 1 %), 183 ([M⁺ - CH₃], 1 %), 155, ([M⁺ - C₂H₃O], 8 %), 141 ([M⁺ - C₃H₅O], 21 %), 127 ([M⁺ - C₄H₆O], 81 %), 114 ([C₆H₁₀O₂⁺] 81 %), 71 ([C₅H₁₁⁺], 100 %).

Messung von: ¹H, (H,H)COSY, hsqc, hmbc, ¹³C, dept NMR
¹H NMR (400 MHz, CDCl₃): δ = 3.08-2.98 (m, 1H, CH₃COCH), 2.97 (dd, *J* = 17.2 Hz, 9.9 Hz, 1H, CH₃COCH₂), 2.44 (dd, *J* = 17.2 Hz, 2.9 Hz, 1 H, CH₃COCH₂), 2.25 (s, 3H, COCH₃), 2.16 (s, 3H, COCH₃), 1.63-1.52 (m, 2H, CH₃COCHCH₂), 1.36-1.21 (m, 8H, CH₃(CH₂)₄), 0.89 (t, *J* = 7.0 Hz, CH₂CH₃).

¹³C NMR (100.6 MHz, CDCl₃): δ = 211.5 (CO), 207.4 (CO), 46.8 (CH₃COCH), 44.7 (CH₃COCH₂), 31.5, 31.2 (CH₃COCHCH₂), 29.9 (COCH₃), 29.7 (COCH₃), 29.2, 27.0, 22.5, 14.0 (CH₂CH₃).

Chirale GC (FS-Lipodex D, isokratisch, 90 °C, nur 1 Peak nach 187.1 min, 95 °C, nur 1 Peak nach 139.9 min, *ee* > 99 %).

[α]_{D}²⁰ = - 54 (α = - 0.215, *c* = 4 mg/ mL, Chloroform).

### Beispiel 4: PigD katalysierte Umsetzung von (E)-4-Phenylbut-3-en-2-on zu 3-Phenylhexan-2,5-dion.

Die PigD katalysierte Umsetzung von (*E*)-4-Phenylbut-3-en-2-on zu 3-Phenylhexan-2,5-dion ist in Abbildung 13 dargestellt. Reaktionsansatz und Analyse des Produkts: 33.7 mg

Protein; 9 % 3-Phenylhexan-2,5-dion im Rohprodukt (GCMS); Nach Reinigung über Säulenchromatographie (R_{f}(Cyclohexan/ Ethylacetat = 5:1) = 0.28): 3.4 mg 3-Phenylhexan-2,5-dion (7.4 % Ausbeute).

GCMS Rₜ = 9.26 min. MS (70 eV, EI); *m*/*z*: 190 ([M⁺], 75 %), 172 ([M⁺ - H₂O], 19 %), 148 ([M⁺ - C₂H₂O], 100 %), 133 ([M⁴ - C₃H₅O], 28 %), 115 (9 %), 105 ([C₇H₅O⁺], 69 %), 91 (19 %), 77 ([C₆H₅⁺], 31 %).

Messung von: ¹H, (H,H)COSY, hsqc, hmbc, ¹³C NMR
¹H NMR (400 MHz, CDCl₃): δ = 7.38-7.27 (m, 3H, Ar), 7.24-7.19 (m, 2H, Ar), 4.24 (dd, *J* = 10.2 Hz, 3.8 Hz, 1H, ArCH), 3.46 (dd, *J* = 18.0 Hz, 10.2 Hz, 1H, CH₂), 2.59 (dd, *J* = 18.0 Hz, 3.8 Hz, 1H, CH₂), 2.19 (s, 3H, COCH₃), 2.14 (s, 3H, COCH₃).

¹³C NMR (100.6 MHz, CDCl₃): δ = 207.1 (CO), 206.8 (CO), 137.8 (Ar, q), 129.1 (Ar, 2C), 128.2 (Ar, 2C), 127.5 (Ar), 53.9 (CH), 46.4 (CH₂), 29.9 (CH₃), 28.9 (CH₃).

CD(CH₃CN):
λ(nm) (Δε) = 190(0.9), 197(11.5), 210(3.6), 217(4.4), 234(-0.1), 287(-6.3).
[α]_{D}²⁰ = - 300 (α = - 0.300, *c* = 1.0 mg/ mL, Chloroform).

Chirale HPLC-DAD
(HPLC Methode: Chiracel OD-H, 15 °C, 0.70 mL·min⁻¹, n-Hexan/ 2-Propanol = 97:3) Rₜ = 29.3 min (*ent*) und 44.2 min (Hauptenantiomer). *ee* = 91 % (nach Säulenchromatographie).

Chirale LCMS
(HPLC Methode: Chiracel OD-H, 20 °C, 0.70 mL·min⁻¹, n-Hexan/ 2-Propanol = 97:3); Rₜ = 15.2 min (*ent*) und 21.3 min (Hauptenantiomer), Extrahierte Masse + Q1: 191.0 amu ; ee = 95 % (direkt extrahiert nach 16 Stunden bei 30 °C).

### Beispiel 5: PigD katalysierte Umsetzung von (E)-4-(4-Chlorphenyl)but-3-en-2-on zu 3-(4-Chlorphenyl)hexan-2,5-dion.

Die PigD katalysierte Umsetzung von (*E*)-4-(4-Chlorphenyl)but-3-en-2-on zu 3-(4-Chlorphenyl)hexan-2,5-dion ist in Abbildung 14 dargestellt. Reaktionsansatz und Analyse des Produkts: 11.3 mg Protein; 18 % (*E*)-4-(4-Chlorphenyl)but-3-en-2-on im Rohprodukt (GCMS); Nach Reinigung über Säulenchromatographie (R_{f}(Cyclohexan/ Ethylacetat = 5:1) = 0.20): 7 mg 3-(4-Chlorphenyl)hexan-2,5-dion (13 % Ausbeute).

GCMS Rₜ = 10.44 min. MS (70 eV, EI); *m*/*z*: 226 (33 %), 224 ([M⁺], 100 %), 208 (2 %), 206 ([M⁺ - H₂O], 6 %), 183 (25 %), 182 (75 %), 181 ([M⁺ - C₂H₂O], 50 %), 169 (5 %), 167 ([M⁺ - C₃H₅O], 15 %), 147 (34 %), 140 (10 %), 138 ([M⁺ - C₂H₂O - C₂H₃O], 29 %), 127 (8 %), 125 ([C₇H₆Cl⁺], 25 %), 115 (6 %), 103 ([M⁺ - C₂H₂O - C₂H₃O - Cl], 40 %), 77 ([C₆H₅⁺], 25 %).

Messung von: ¹H, hsqc, ¹³C NMR
¹H NMR (400 MHz, CDCl₃): δ = 7.35-7.30 (m, 2H, Ar), 7.19-7.13 (m, 2H, Ar), 4.22 (dd, *J* = 10.0 Hz, 4.1 Hz, 1H, ArCH), 3.42 (dd, *J* = 18.0 Hz, 10.0 Hz, 1H, CH₂), 2.57 (dd, *J* = 18.0 Hz, 4.1 Hz, 1H, CH₂), 2.18 (s, 3H, COCH₃), 2.14 (s, 3H, COCH₃).

¹³C NMR (100.6 MHz, CDCl₃): δ = 206.6 (CO), 206.3 (CO), 136.2 (Ar, q), 133.6 (Ar, q), 129.5 (Ar, 2C), 129.3 (Ar, 2C), 53.1 (CH), 46.3 (CH₂), 29.9 (CH₃), 28.9 (CH₃).

CD(CH₃CN): λ(nm) (Δε) = 213(3.1), 225(6.7), 246(-0.7), 287(-8.9).

[α]_{D}²⁰ = - 314 (α = -1.571, c = 5 mg/ mL, Chloroform).

Chirale HPLC
(HPLC Methode: Chiracel OD-H, 15 °C, 0.50 mL·min⁻¹, n-Hexan/ 2-Propanol = 99:1) Rₜ = 45.3 min, nur 1 Peak (ee > 98 %)

### Beispiel 6: PigD katalysierte Umsetzung von (E)-4-(Furan-2-yl)but-3-en-2-on zu 3-(Furan-2-yl)hexan-2,5-dion.

Die PigD katalysierte Umsetzung von (*E*)-4-(Furan-2-yl)but-3-en-2-on zu 3-(Furan-2-yl)hexan-2,5-dion ist in Abbildung 15 dargestellt. Reaktionsansatz und Analyse des Produkts: 20 mg Protein; 12 % 3-(Furan-2-yl)hexan-2,5-dion im Rohprodukt (GCMS); Nach Reinigung über Säulenchromatographie (R_{f}(Cyclohexan/ Ethylacetat = 5:1) = 0.28): 3.3 mg 3-(Furan-2-yl)hexan-2,5-dion (6 % Ausbeute).

GCMS Rₜ = 8.03 min. MS (70 eV, El); *m*/*z*: 180 ([M⁺], 100 %), 162 ([M⁺- H₂O], 5 %), 137 ([M⁺ - C₂H₃O], 88 %), 121 (5 %), 109 (9 %), 95 (63 %), 81 (21 %), 65 (29 %).

Messung von: ¹H, hsqc, ¹³C NMR
¹H NMR (400 MHz, CDCl₃): δ = 7.34 (dd, *J* = 1.9 Hz, 0.8 Hz, 1H, Furanyl), 6.35 (dd, = 3.2 Hz, 1.9 Hz, 1H, Furanyl), 6.17 (d, *J* = 3.2 Hz, 1H, Furanyl), 4.36 (dd, *J* = 9.8 Hz, 4.2 Hz, 1H, CH), 3.42 (dd, J = 18.0 Hz, 9.8 Hz, 1H, CH₂), 2.68 (dd, *J* = 18.0 Hz, 4.2 Hz, 1H, CH₂), 2.210 (s, 3H, COCH₃), 2.213 (s, 3H, COCH₃).

¹³C NMR (100.6 MHz, CDCl₃): δ = 206.1 (CO), 204.5 (CO), 171.1 (Furanyl), 142.3 (Furanyl), 110.6 (Furanyl), 107.3 (Furanyl), 47.2(CH), 43.3 (CH₂), 29.8 (CH₃), 28.7 (CH₃).

### Beispiel 7: Bestimmung des Enantiomerenüberschusses

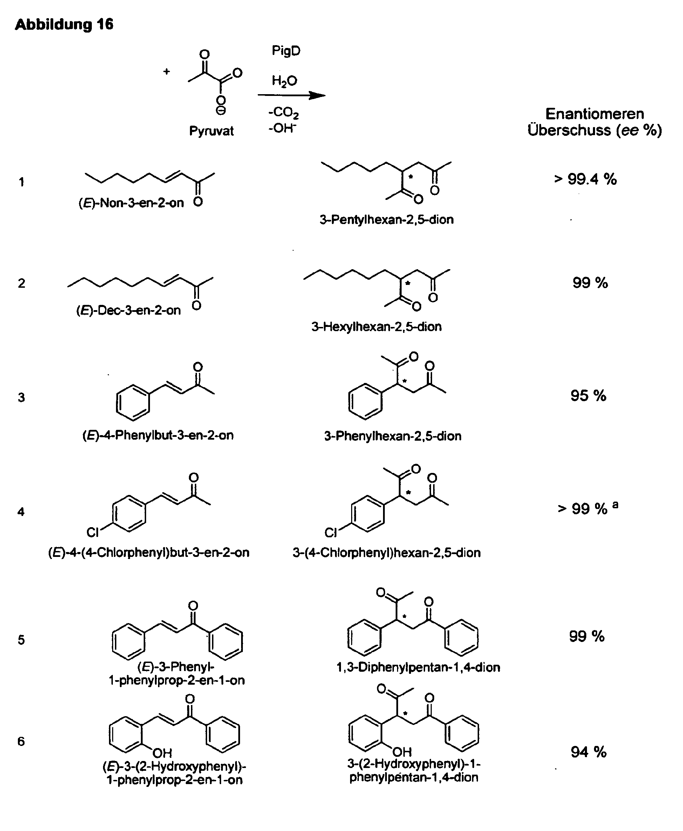

Der Enantiomerenüberschuss der durch die PigD-Katalyse entstandenen Produkte wurde über chirale GC-FID (Gaschromatographische Trennung mit Flammenionisationsdetektor), chirale HPLC-DAD (Hochdruckflüssigkeitschromatographie mit Diodenarray Detektor) bzw. chirale LCMS (Hochdruckflüssigkeitschromatographie mit massenspektrometrischer Detektion) mit Photospray-lonenquelle bestimmt (siehe Abbildung 16).

### Beispiel 8: Chemische Synthese der Referenzsubstanz 3-Pentylhexan-2,5-dion

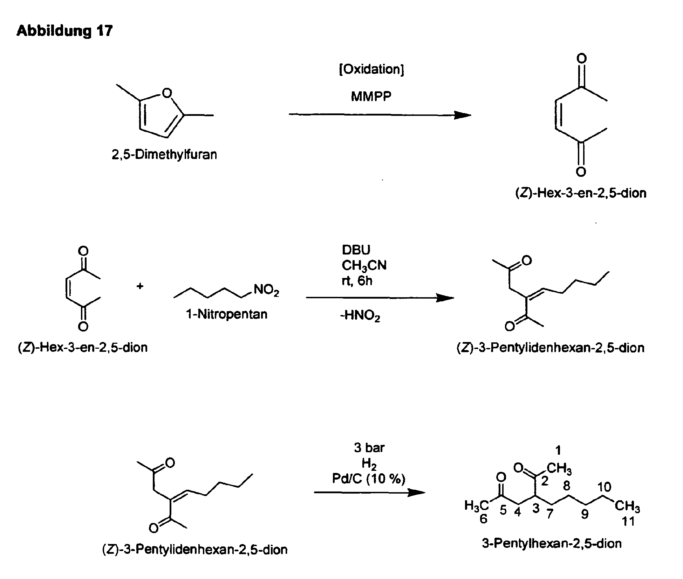

Um das Additionsprodukt aus Beispiel 2 analysieren zu können, wurde durch chemische Synthese racemisches 3-Pentylhexan-2,5-dion (siehe Abbildung 17) als Referenz hergestellt (Dominguez et al., Tetrahedron Letters 1990, 31, 7669; Ballini et al., Synthesis-Stuttgart 2001, 2003).

Analysendaten von 3-Pentylhexan-2,5-dion:
GCMS Rₜ = 8.49 min. MS (70 eV, El); *m*/*z*: 184 ([M⁺], 1 %), 169 ([M⁺ - CH₃], 3 %), 141 ([M⁺ - C₂H₃O], 10 %), 127 ([M⁺ - C₃H₅O], 25 %), 114 ([C₆H₁₀O₂⁺], 80 %), 71 ([C₅H₁₁⁺], 100 %).

Messung von: ¹H-, ¹³C-, hsqc- and (H,H)-cosy NMR.
¹H NMR (400 MHz, CDCl₃): δ = 3.07-2.98 (m, 1 H, 3), 2.97 (dd, *J* = 17.3 Hz, *J* = 9.9 Hz, 1 H, 4), 2.44 (dd, *J* = 17.3 Hz, *J* = 3.0 Hz, 1 H, 4), 2.24 (s, 3 H, 1), 2.15 (s, 3 H, 6), 1.52-1.67 (m, 2 H, 7), 1.20-1.35 (m, 6 H, 8, 9, 10), 0.89 (t, 3 H, 11).

¹³C NMR (77.2 MHz, CDCl₃): δ = 211.6, 207.5, 46.8 (4), 44.7 (3), 31.7 (8), 31.2 (7), 29.9 (6), 29.7 (1), 26.7 (9), 22.4 (10), 13.9 (11).

Chirale GC (Racemat) ; (FS-Lipodex D, isokratisch, 90 °C) Rₜ = 98.9 min, 102.6 min.

### Zu Gleichung 1 aus Abbildung 16:

Chirale Gaschromatographie
(FS-Lipodex D, isokratisch, 90 °C),
Rₜ = 98.9 min (Hauptenantiomer), 102.6 min (*ent*).
*ee* = 99.4 % (nach Säulenchromatographie)
Durch die Säulenchromatographie und/ oder Temperaturen über 20 °C kommt es zu einer leichten Racemisierung aufgrund von Produktinstabilität. Es können also in diesem Fall schon 0.3 % des entgegengesetzten Enantiomers mittels chiraler GC detektiert werden. Bei Extraktion einer Probe mit Ethylacetat ohne Reinigung nach 40 Stunden Reaktionszeit wird über die entwickelte chirale GC Methode kein entgegengesetztes Enantiomer detektiert. Der Enantiomerenüberschuss des enzymatisch gebildeten Produktes beträgt somit: ee > 99.4 %.
Der Enantiomerenüberschuss wurde über chirale Gaschromathographie mit dem chemisch synthetisierten Racemat als Referenz ermittelt.

### Zu Gleichung 2 aus Abbildung 16:

Chirale Gaschromathographie
(FS-Lipodex D, isokratisch, 90 °C) nur 1 Peak nach 187.1 min,
(FS-Lipodex D, isokratisch, 95 °C) nur 1 Peak nach 139.9 min,
Das Standard Detektionslimit der chiralen GC liegt, wenn nichts anderes nachgewiesen wurde (siehe Gleichung 1) bei 0.5 %.
Da über verschiedene GC-Analysenmethoden nur ein Peak detektiert wurde, wurde schlussgefolgert, dass hier der Enantiomerenüberschuss des enzymatisch gebildeten Produktes bei ee > 99 % liegt.

Chirale LCMS (MS (+Q1): 199 [M+1], 181 [M+1-H₂O]),
(HPLC Methode: Chiralpak AD, 20 °C, 0.75 mL·min⁻¹, n-Hexan/ 2-Propanol = 97:3)
Rₜ = 10.4 min (Hauptenantiomer), 20.4 min (*ent*),
Enantiomerenüberschuss des enzymatisch gebildeten Produktes: ee = 99.1 %.

### Zu Gleichung 3 aus Abbildung 16:

Chirale HPLC-DAD
(HPLC Methode: Chiracel OD-H, 15 °C, 0.70 mL·min⁻¹, n-Hexan/ 2-Propanol = 97:3),
Rₜ = 29.3 min (ent) und 44.2 min (Hauptenantiomer).
*ee* = 91 % (nach Säulenchromatographie)

Chirale LCMS (Extrahierte Masse + Q1: 191.0 amu),
(HPLC Methode: Chiracel OD-H, 20 °C, 0.70 mL·min⁻¹, n-Hexan/ 2-Propanol = 97:3),
Rₜ = 15.2 min (ent) und 21.3 min (Hauptenantiomer),
Enantiomerenüberschuss des enzymatisch gebildeten Produktes: ee = 95 % (direkt extrahiert nach 16 Stunden bei 30 °C).

### Zu Gleichung 4 aus Abbildung 16:

Chirale HPLC-DAD
(HPLC Methode: Chiracel OD-H, 15 °C, 0.50 mL·min⁻¹, n-Hexan/ 2-Propanol = 99:1),
Rₜ = 45.3 min, nur 1 Peak.
(auch über folgende HPLC Analysenmethoden immer nur 1 Peak detektiert: Chiracel OD-H, 15 °C. 0.70 mL·min⁻¹, n-Hexan/ 2-Propanol = 99:1, Chiracel OD-H, 15 °C, 0.50 mL·min⁻¹, n-Hexan/ 2-Propanol = 97:3, Chiracel OD-H, 15 °C, 0.50 mL·min⁻¹, n-Hexan = 100, Chiralpak AD, 20 °C, 0.75 mL·min⁻¹, n-Hexan/ 2-Propanot = 95:5, Chiralpak AD, 20 °C, 0.75 mL·min⁻¹, n-Hexan/ 2-Propanol = 97:3, Chiralpak AD, 20 °C, 0.75 mL·min⁻¹, n-Hexan/ 2-Propanol = 99:1, Chiralpak AD, 20 °C, 0.75 mL·min⁻¹, n-Hexan = 100, Chiracel OB, 20 °C, 0.75 mL·min⁻¹, n-Hexan/ 2-Propanol = 90:10, Chiracel OB, 20 °C, 0.75 mL·min⁻¹, n-Hexan/ 2-Propanol = 95:5, Chiracel OB, 20 °C, 0.75 mL·min⁻¹, n-Hexan/ 2-Propanol = 97:3.

Chirale LCMS (MS (+Q1): 225 [M+1], 207 [M+1-H₂O]),
(HPLC Methode: Chiralpak AD, 20 °C, , 0.75 mL·min⁻¹, n-Hexan/ 2-Propanol = 95:5),
Rₜ = 11.0 min, nur 1 Peak.

Das Standard Detektionslimit der chiralen LCMS mit Photosprayionenquelle liegt, wenn nichts anderes nachgewiesen wurde bei, 0.5 %.
Da über verschiedene Analysenmethoden nur ein Peak detektiert wurde, wurde schlussgefolgert, dass hier der Enantiomerenüberschuss des enzymatisch gebildeten Produktes bei ee > 99 % liegt.

### Zu Gleichung 5 aus Abbildung 16:

Chirale LCMS (MS (+Q1): 253 [M+1], 235 [M+1-H₂O]),
(HPLC Methode: Chiralpak AD, 20 °C, 0.75 mL·min⁻¹, n-Hexan/ 2-Propanol = 95:5)
Rₜ = 17.6 min (*ent*) 19.5 min (Hauptenantiomer),
Enantiomerenüberschuss des enzymatisch gebildeten Produktes: ee = 99 %.

### Zu Gleichung 6 aus Abbildung 16:

Chirale HPLC
(HPLC Methode: Chiracel OD-H, 25 °C, 0.75 mL·min⁻¹, n-Hexan/ 2-Propanol = 96:4)
Rₜ = 58.8 min (Hauptenantiomer), 65.0 min (*ent*)*.*
*ee* = 81 % (nach Säulenchromatographie)
Enantiomerenüberschuss des enzymatisch gebildeten Produktes: ee = 94 % (direkt extrahiert nach 16 Stunden bei 30 °C).

### Beispiel 9: Bestimmung der absoluten Konfiguration über chemisch synthetisiertes (S)-3-Phenylhexan-2,5-dion als Vergleich

Zur Bestimmung der absoluten Konfiguration des Produkts aus Beispiel 4 wurde eine chemische Synthese von (S)-3-Phenylhexan-2,5-dion durchgeführt (Clark et al., Journal of Organic Chemistry 1970, 35, 1114).

Aus (*S*)-2-Phenylbemsteinsäure wurde mit Methyllithium in Diethylether (*S*)-3-Phenylhexan-2,5-dion synthetisiert. Die analytischen Daten stimmen bis auf Drehwert und CD mit dem über PigD-Katalyse entstandenen Produkt (Abbildungen 10 und 16) überein.

R_{f}(SiO₂. Cyclohexan: Ethylacetat = 10:1) = 0.23.

GCMS Rₜ = 9.26 min. MS (70 eV, EI); *m*/*z*: 190 ([M⁺], 75 %), 172 ([M⁺ - H₂O], 19 %), 148 ([M⁺ - C₂H₂O], 100 %), 133 ([M⁺ - C₃H₅O], 28 %), 115 (9 %), 105 ([C₇H₅O⁺], 69 %), 91 (19 %), 77 ([C₆H₅⁺], 31 %).

¹H NMR (400 MHz, CDCl₃) δ = 7.38-7.27 (m, 3H, Ar), 7.24-7.19 (m, 2H, Ar), 4.24 (dd, *J* = 10.2 Hz, 3.8 Hz, 1H, ArCH), 3.46 (dd, *J* = 18.0 Hz, 10.2 Hz, 1H, CH₂), 2.59 (dd, *J* = 18.0 Hz, 3.8 Hz, 1H, CH₂), 2.19 (s, 3H, COCH₃), 2.14 (s, 3H, COCH₃).

¹³C NMR (100.6 MHz, CDCl₃) δ = 207.1 (CO), 206.8 (CO), 137.8 (Ar, q), 129.1 (Ar, 2C), 128.2 (Ar, 2C), 127.5 (Ar), 53.9 (CH), 46.4 (CH₂), 29.9 (CH₃), 28.9 (CH₃).

Chirale HPLC-DAD
(HPLC Methode: Chiracel OD-H, 15 °C, 0.70 mL·min⁻¹, n-Hexan/ 2-Propanol = 97:3)
Rₜ = 29.3 min (Hauptenantiomer), 44.2 min (*ent*)*. ee* = 75 % (nach Säulenchromatographie)

Chirale LCMS (Extrahierte Masse + Q1: 191.0 amu)
(HPLC Methode: Chiracel OD-H, 20 °C, 0.70 mL·mim⁻¹, n-Hexan/ 2-Propanol = 97:3); Rₜ = 15.2 min (Hauptenantiomer) und 21.3 min (*ent*)*.*
*ee* = 75 % (nach Säulenchromatographie)

CD(CH₃CN):
λ(nm) (Δε) = 197(-8.7), 209(-2.9), 217(-3.5), 239(0.1), 286(5.1).
[α]_{D}²⁰ = + 173 (α = + 0.104, c = 0.6 mg/ mL, Chloroform).

Optische Rotation und CD (Circular Dichroismus).

Drehwert der Produkte durch PigD Katalyse:
3-Hexylhexan-2,5-dion (Abbildung 6):
   [α]_{D}²⁰ = - 54 (α = - 0.215, *c* = 4 mg/ mL, Chloroform).
3-Phenylhexan-2,5-dion (Abbildung 7):
   [α]_{D}²⁰ = - 300 (α = - 0.300, *c* = 1.0 mg/ mL, Chloroform).
3-(4-Chlorphenyl)hexan-2,5-dion (Abbildung 7):
   [α]_{D}²⁰ = - 314 (α = - 1.571, *c* = 5 mg/ mL, Chloroform).
1,3-Diphenylpentan-1,4-dion (Abbildung 7):
   [α]_{D}²⁵ = - 179 (α = - 0.357, *c* = 2 mg/ mL, Chloroform).
Drehwert von (*S*)-3-Phenylhexan-2,5-dion (Abbildung 7):
   [α]_{D}²⁰ = + 173 (α = + 0.104, *c* = 0.6 mg/ mL, Chloroform).

### Absolute Konfiguration

Über Trennung an chiraler Phase mittels HPLC/MS konnte gezeigt werden, dass durch PigD Katalyse das entgegengesetzte Enantiomer zum chemisch synthetisierten (*S*)-3-Phenylhexan-2,5-dion in sehr großem Enantiomerenüberschuss gebildet wird. Die Drehwerte und CD Spektren des chemisch synthetisierten (*S*)-3-Phenylhexan-2,5-dion wurden mit denen der Produkte verglichen, die durch PigD-Katalyse erhalten wurden und es wurden für die chiroptischen Eigenschaften spiegelbildliche Ergebnisse erhalten. Diese Informationen führen zu der Schlussfolgerung, dass die asymmetrische intermolekulare Stetter-Reaktion, katalysiert durch das Enzym PigD, Produkte mit R-Konfiguration liefert, wenn R³ ein Aromat (R³ über einen sp² hybridisierten Kohlenstoff gebunden) ist. Im Falle eines Aliphaten (R³ über einen sp³ hybridisierten Kohlenstoff gebunden) kehrt sich die Konfigurationsbestimmung formal um.

### Beispiel 10: Herstellung des PigD-Proteins

### Klonierung:

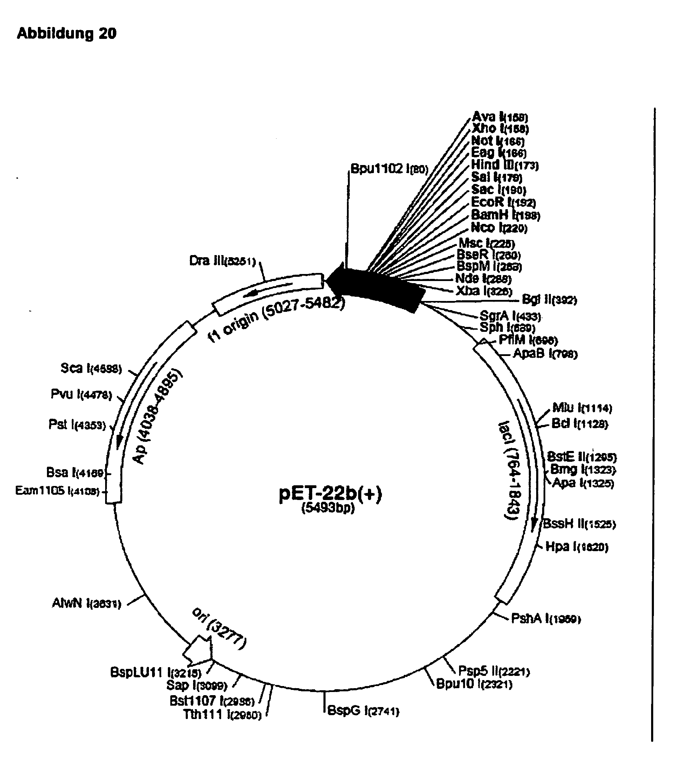

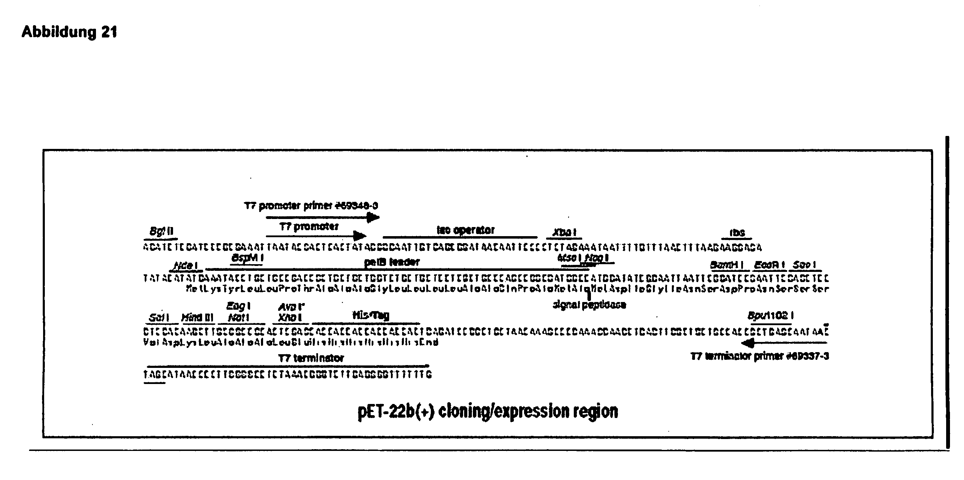

Das Ausgangsmaterial für die Arbeiten war die genomische DNA aus *Serratia marcescens.* Mittels PCR (Polymerase Kettenreaktion) wurde die DNA, die dem Protein PigD aus *Serratia marcescens* entspricht, vervielfältigt. Durch die dabei verwendeten Primer wurde außerdem am 5'-Ende eine *Nde*I-, sowie am 3'-Ende eine *Xh*oI-Restriktionsschnittstelle generiert. Der Vektor pET22b(+) (Abbildung 20) besitzt in seiner Multi Cloning Site (MCS) (Abbildung 21) diese beiden Schnittstellen. Sowohl der Vektor als auch das jeweilige PCR-Produkt wurden dann mit den beiden Restriktionsenzymen verdaut und im Anschluss mit T4 DNA-Ligase ligiert.

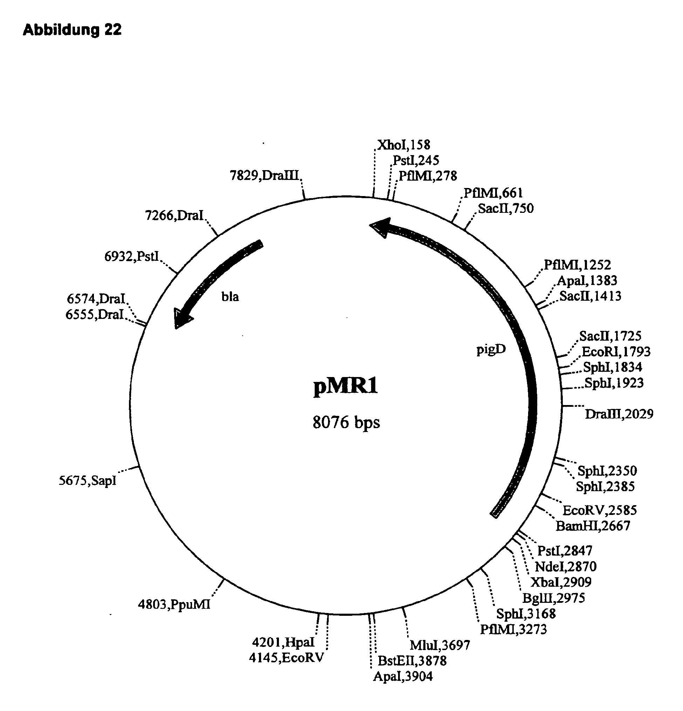

Mit dem so erhaltenen Vektor (Abbildung 22) wurden *E. coli* DH5α zur Vektorproduktion und *E. coli* XL1blue-, bzw. *E*. *coli* BL21(DE3)plysS-, *E. coli* BL21(DE3)*CP- und *E. coli* BL21(DE3)-Zellen zur Expression transformiert. Auf diese Weise wurde ein Fusionsprotein von PigD aus *Serratia marcescens* als C-terminal His-Tag tragendes Protein erhalten. Die erhaltene Aminosäuresequenz ist als Aminosäuresequenz SEQ ID NO: 1 in dem Sequenzprotokoll abgebildet

### Expression:

Die beste Expression wurde in *E. coli* BL21 (DE3) Zellen erhalten. Die Zellen wurden in LB-Medium (100 µg/ml Ampicillin) bei 37 °C und 120 rpm im Schüttelkolben inkubiert und nach IPTG-Zugabe (0.4 mM) über Nacht bei 29 °C exprimiert. Des Weiteren wurde ein Fermenterlauf durchgeführt, der eine niedrigere Expression aufwies.

### Protein-Reinigung:

Die Proteinreinigung wurde über Immobilisierte Metallionen Affinitätschromatographie durchgeführt. Es wurden sowohl Talon^{®}beads als auch Nickel-NTA-Sepharose verwendet.

## Patentansprüche

1. Verfahren zur Herstellung von Verbindungen der Formel II 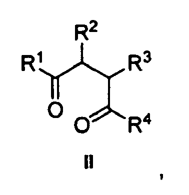 **dadurch gekennzeichnet, dass** Verbindungen der Formel I 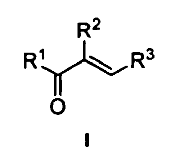 mit 2-Oxoalkanoaten oder 2-Oxocarbonsäuren der Formel R⁴C(O)COO- bzw. R⁴C(O)COOH umgesetzt werden,
worin R¹ einen geradkettigen oder verzweigten C₁-C₁₀-Alkylrest oder Aromaten,
R² einen Wasserstoff, C₁-C₁₀-Alkylrest oder Aromaten,
R³ einen geradkettigen oder verzweigten C₁₋₁₀-Alkylrest, einen Heterocyclus oder einen Aromaten, wobei der C₁₋₁₀-Alkylrest wiederum einen Heterocyclus oder einen Aromaten aufweisen kann, und wobei der Heterocyclus oder der Aromat mit Methoxygruppen, Halogenen oder Hydroxygruppen substituiert sein kann, und
R⁴ einen geradkettigen oder verzweigten C₁-C₁₀-Alkylrest darstellt, und
worin das PigD-Protein als Katalysator verwendet wird.

2. Verfahren gemäß Anspruch 1, **dadurch gekennzeichnet, dass** R¹ eine Methylgruppe oder einen Aromaten, R² einen Wasserstoff oder einen Aromaten, und R³ eine C₁₋₁₀-Alkylgruppe, einen Heterocyclus oder einen Aromaten darstellt, wobei der Heterocyclus oder der Aromat mit Methoxygruppen, Halogenen oder Hydroxygruppen substituiert sein kann, und worin R⁴ einen C₁-C₁₀-Kohlenwasserstoff darstellen kann.

3. Verfahren gemäß einem der vorangegangenen Ansprüche, **dadurch gekennzeichnet, dass** die 1,4-Additionen enantioselektiv mit einem Enantiomerenüberschuss von mehr als 80 % ee erfolgen.

4. Verfahren gemäß einem der vorangegangenen Ansprüche, **dadurch gekennzeichnet, dass** geradkettige, gesättigte 2-Oxoalkanoate oder 2-Oxocarbonsäuren mit 1-10 Kohlenstoffatomen als Donorsubstrate verwendet werden.

5. Verfahren gemäß einem der vorangegangenen Ansprüche, **dadurch gekennzeichnet, dass** Pyruvat bzw. Brenztraubensäure oder 2-Oxobutanoat bzw. 2-Oxobuttersäure als Donorsubstrate verwendet werden und R₄ gleich Methyl oder Ethyl ist.

6. Verwendung des PigD-Proteins zur Katalyse von 1,4-Additionen von 2-Oxo-alkanoaten/ -carbonsäuren an α,β-ungesättigte Ketone der Formel I, 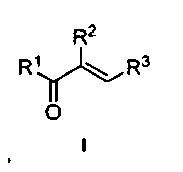 worin R¹ einen geradkettigen oder verzweigten C₁-C₁₀-Alkylrest oder einen Aromaten,
R² einen Wasserstoff, C₁-C₁₀-Alkylrest oder einen Aromaten, und
R³ einen geradkettigen oder verzweigten C₁₋₁₀-Alkylrest, einen Heterocyclus oder einen Aromaten darstellt, wobei die C₁₋₁₀-Alkylgruppe wiederum einen Heterocyclus oder einen Aromaten aufweisen kann, und wobei der Heterocyclus oder der Aromat mit Methoxygruppen, Halogenen oder Hydroxygruppen substituiert sein können, und wobei die 1,4-Additionen unter CO₂-Abspaltung erfolgen.

7. Verwendung des PigD-Proteins gemäß Anspruch 6, **dadurch gekennzeichnet, dass** Pyruvat bzw. Brenztraubensäure oder 2-Oxobutanoat bzw. 2-Oxobuttersäure als 2-Oxoalkanoat bzw. 2-Oxocarbonsäure verwendet wird.

8. Verwendung des PigD-Proteins gemäß einem der Ansprüche 6 oder 7, **dadurch gekennzeichnet, dass** die 1,4-Additionen enantioselektiv mit einem Enantiomerenüberschuss der Additionsprodukte von mehr als 80 % ee erfolgen.

9. Verwendung des PigD-Proteins gemäß einem der Ansprüche 6 bis 8, **dadurch gekennzeichnet, dass** aliphatische, aromatische und heterocyclische α,β-ungesättigte Ketone gemäß der Formel I eingesetzt werden, worin
R¹ eine Methylgruppe oder einen Aromaten, R² ein Wasserstoffatom, C₁-C₁₀-Alkylrest oder einen Aromaten, und R³ eine C₁₋₁₀-Alkylgruppe, einen Heterocyclus oder einen Aromaten, wobei der Heterocyclus und der Aromat mit Methoxygruppen, Halogenen oder Hydroxygruppen substituiert sein kann, darstellt.

## Claims

1. A process for preparing compounds of the formula II 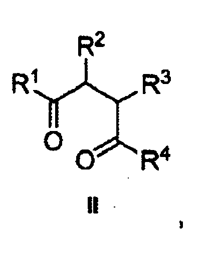 **characterized in that** compounds of the formula I 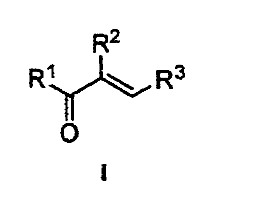 are reacted with 2-oxoalkanoates or 2-oxocarboxylic acids of the formula R⁴C(O)Coo⁻ or R⁴C(O)COOH,
in which R¹ is a straight-chain or branched C₁-C₁₀-alkyl radical or aromatic,
R² is a hydrogen, C₁-C₁₀-alkyl radical or aromatic,
R³ is a straight-chain or branched C₁-C₁₀-alkyl radical, a heterocycle or an aromatic, where the C₁-C₁₀-alkyl radical may in turn have a heterocycle or an aromatic, and where the heterocycle or the aromatic may be substituted by methoxy groups, halogens or hydroxyl groups, and
R⁴ is a straight-chain or branched C₁-C₁₀-alkyl radical and
in which the PigD protein is used as a catalyst.

2. The process as claimed in claim 1, **characterized in that** R¹ is a methyl group or an aromatic, R² is a hydrogen or an aromatic, and R³ is a C₁-C₁₀-alkyl group, a heterocycle or an aromatic, where the heterocycle or the aromatic may be substituted by methoxy groups, halogens or hydroxyl groups, and in which R⁴ may be a C₁-C₁₀-hydrocarbon.

3. The process as claimed in any of the preceding claims, **characterized in that** the 1,4 additions are effected enantioselectively with an enantiomeric excess of more than 80% ee.

4. The process as claimed in any of the preceding claims, **characterized in that** straight-chain, saturated 2-oxoalkanoates or 2-oxocarboxylic acids with 1-10 carbon atoms are used as donor substrates.

5. The process as claimed in any of the preceding claims, **characterized in that** pyruvate or pyruvic acid or 2-oxobutanoate or 2-oxybutyric acid are used as donor substrates, and R⁴ is methyl or ethyl.

6. The use of the PigD protein for catalysis of 1,4 additions of 2-oxoalkanoates/-carboxylic acids onto α,β-unsaturated ketones of the formula I 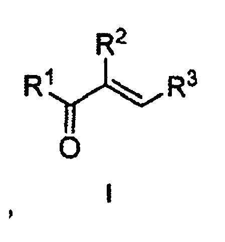
in which R¹ is a straight-chain or branched C₁-C₁₀-alkyl radical or an aromatic,
R² is a hydrogen, C₁-C₁₀-alkyl radical or an aromatic, and
R³ is a straight-chain or branched C₁-C₁₀-alkyl radical, a heterocycle or an aromatic, where the C₁-C₁₀-alkyl radical may in turn have a heterocycle or an aromatic, and where the heterocycle or the aromatic may be substituted by methoxy groups, halogens or hydroxyl groups, and where the 1,4 additions are effected with CO₂ elimination.

7. The use of the PigD protein as claimed in claim 6, **characterized in that** pyruvate or pyruvic acid or 2-oxobutanoate or 2-oxobutyric acid is used as the 2-oxoalkanoate or 2-oxocarboxylic acid.

8. The use of the PigD protein as claimed in any of claims 6 and 7, **characterized in that** the 1,4 additions are effected enantioselectively with an enantiomeric excess of the addition products of more than 80% ee.

9. The use of the PigD protein as claimed in any of claims 6 to 8, **characterized in that** aliphatic, aromatic and heterocyclic α,β-unsaturated ketones of the formula I are used, in which
R¹ is a methyl group or an aromatic, R² is a hydrogen atom, C₁-C₁₀ alkyl radical or an aromatic, and R³ is a C₁-C₁₀-alkyl group, a heterocycle or an aromatic, where the heterocycle and the aromatic may be substituted by methoxy groups, halogens or hydroxyl groups.

## Revendications

1. Procédé de fabrication de composés de formule II : 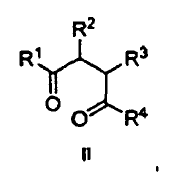 **caractérisé en ce que** l'on fait réagir des composés de formule I : 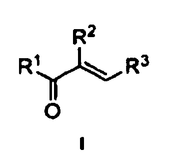 avec des 2-oxoalcanoates ou des acides 2-oxocarboxyliques de formule R⁴C(O)C00⁻ ou R⁴C(O)COOH,
dans laquelle R¹ représente un radical alkyle en C₁-C₁₀ linéaire ou ramifié ou un composé aromatique,
R² représente l' hydrogène, un radical alkyle en C₁-C₁₀ ou un composé aromatique,
R³ représente un radical alkyle en C₁-C₁₀ linéaire ou ramifié, un hétérocycle ou un composé aromatique, le radical alkyle en C₁-C₁₀ pouvant à son tour présenter un hétérocycle ou un composé aromatique et l'hétérocycle ou le composé aromatique pouvant être substitué par des groupements méthoxy, par des halogènes ou par des groupements hydroxyle, et
R⁴ représente un radical alkyle en C₁-C₁₀ linéaire ou ramifié, et
dans lequel on utilise la protéine PigD comme catalyseur.

2. Procédé selon la revendication 1, **caractérisé en ce que** R¹ représente un groupement méthyle ou un composé aromatique, R² représente l'hydrogène ou un composé aromatique et R³ représente un groupement alkyle en C₁-C₁₀, un hétérocycle ou un composé aromatique, dans lequel l'hétérocycle ou le composé aromatique peut être substitué par des groupements méthoxy, par des halogènes ou par des groupements hydroxyle et dans lequel R⁴ peut représenter un hydrocarbure en C₁-C₁₀.

3. Procédé selon l'une quelconque des revendications précédentes, **caractérisé en ce que** les additions de type 1,4 se font de manière énantiosélective avec un excès énantiomérique (ee) supérieur à 80 %.

4. Procédé selon l'une quelconque des revendications précédentes, **caractérisé en ce que** l'on utilise des 2-oxoalcanoates ou des acides 2-oxocarboxyliques linéaires saturés ayant 1 à 10 atomes de carbone comme substrats donneurs.

5. Procédé selon l'une quelconque des revendications précédentes, **caractérisé en ce que** le pyruvate ou l'acide pyruvique ou le 2-oxobutanoate ou l'acide 2-oxobutyrique sont utilisés comme substrats donneurs et le radical R⁴ correspond à un méthyle ou un éthyle.

6. Utilisation de la protéine PigD pour catalyser les réactions d'additions de type 1,4 de 2-oxoalcanoates/acides 2-oxocarboxyliques sur des cétones de type α,β-insaturées de formule I : 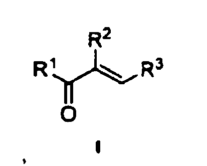 dans laquelle R¹ représente un radical alkyle en C₁-C₁₀ linéaire ou ramifié ou un composé aromatique,
R² représente l'hydrogène, un radical alkyle en C₁-C₁₀ ou un composé aromatique, et
R³ représente un radical alkyle en C₁-C₁₀ linéaire ou ramifié, un hétérocycle ou un composé aromatique, dans lequel le groupement alkyle en C₁-C₁₀ peut présenter à son tour un hétérocycle ou un composé aromatique et dans lequel l'hétérocycle ou le composé aromatique peut être substitué par des groupements méthoxy, par des halogènes ou par des groupements hydroxyle, et les additions de type 1,4 se font avec élimination de CO₂.

7. Utilisation de la protéine PigD selon la revendication 6, **caractérisée en ce que** l'on utilise du pyruvate ou de l'acide pyruvique ou du 2-oxobutanoate ou de l'acide 2-oxobutyrique comme 2-oxoalcanoate ou acide 2-oxocarboxylique.

8. Utilisation de la protéine PigD selon l'une quelconque des revendications 6 ou 7, **caractérisée en ce que** les additions de type 1,4 se font de manière énantiosélective avec un excès énantiomérique (ee) supérieur à 80 %.

9. Utilisation de la protéine PigD selon l'une quelconque des revendications 6 à 8, **caractérisée en ce que** l'on utilise des cétones aliphatiques, aromatiques et hétérocycliques à insaturation de type α,β selon la formule I, dans laquelle
R¹ représente un groupement méthyle ou un composé aromatique, R² représente l'hydrogène, un radical alkyle en C₁-C₁₀ ou un composé aromatique et R³ représente un groupement alkyle en C₁-C₁₀, un hétérocycle ou un composé aromatique, l'hétérocycle et le composé aromatique pouvant être substitués par des groupements méthoxy, par des halogènes ou par des groupements hydroxyle.
